# EUROPEAN PATENT SPECIFICATION

(11) **EP 2 736 425 B1**
(45) Date of publication and mention of the grant of the patent: **05.02.2020**
(21) Application number: 12817411.7
(22) Date of filing: 25.07.2012
(51) Int. Cl.: A61B 17/221, A61F 2/01

(54) **INTRAVASCULAR THROMBOEMBOLECTOMY DEVICE**
INTRAVASKULÄRE THROMBEMBOLEKTOMIEVORRICHTUNG
DISPOSITIF DE THROMBOEMBOLECTOMIE VASCULAIRE

(30) Priority: 26.07.2011 US 201113191306; 06.07.2012 US 201213543657
(43) Date of publication of application: 04.06.2014
(73) Proprietor: Marks, Michael, P., Hillsborough, CA 94010 (US); Que, Like, Livermore, CA 94550 (US)
(72) Inventor: Marks, Michael, P., Hillsborough, CA 94010 (US); Que, Like, Livermore, CA 94550 (US)
(74) Representative: Durán-Corretjer, S.L.P.
(86) International application number: PCT/US2012/048158
(87) International publication number: WO 2013/016435

(56) References cited:
- WO-A1-02/055146
- WO-A1-2009/076482
- JP-A- 2003 033 359
- US-A- 5 011 488
- US-A1- 2002 123 765
- US-A1- 2003 163 158
- US-A1- 2004 260 333
- US-A1- 2009 192 485
- US-A1- 2009 299 393

## Description

### BACKGROUND OF THE INVENTION

### Field of the Invention

The present disclosures are generally related to a device used in a body lumen such as a blood vessel.

### Description of the Related Art

A variety of disease conditions can be caused, at least in part, by blockage or, occlusions or clots of blood vessels. A well-known example of such conditions includes, but is not limited to stroke. Other such conditions include a myocardial infarction, limb ischemia, occlusions or clots of vascular grafts and bypasses, and venous thromboses.

A stroke is often referred as a "brain attack." It often results in rapid and significant loss of brain function due to disturbance in the blood supply to the brain. As a result, inabilities in movement, use of language, vision and many other biological functions may be temporarily or irreversibly impaired. Strokes are either hemorrhagic (due to bleeding) or ischemic (due to inadequate blood supply). The majority of strokes are ischemic. It is estimated that about 700,000 ischemic strokes occur in the United States annually. The major causes of an ischemic stroke include thrombosis (clotting) in a blood vessel supplying the brain or an embolus from another source such as the heart going to a blood vessel supplying the brain. Sometimes a thrombosis occurs where there is a pre-existing stenosis of blood vessels in the brain, usually form atherosclerotic disease.

Treatments for acute ischemic stroke are concentrated on re-establishing blood flow to the brain as quickly as possible. They include the use of a drug such as tissue plasminogen activator (tPA), a thrombolytic agent (clot-busting drug). More recently devices such as the Merci thrombectomy device (Concentric Medical, Mountain View, California) and the Penumbra suction thrombectomy catheter (Penumbra, Inc., Alameda, California) and the Solitaire thrombectomy device (ev3 Neurovascular, Irvine, California) have been approved by the Food and Drug Administration for thrombectomy in acute stroke. These devices do not always achieve complete recanalization. Sometimes they fail to open the vessel at all or may only partially open the vessel. They also may take some time to work, with multiple passes of the devices into the intracranial circulation needed before the vessel is reopened. In addition they may fragment the clot and allow some portion of the clot to go out more distally in the cerebral circulation. There is a need for devices with high rates of complete recanalization, with complete or partial clot capture, performed in a more rapid manner. US 2009/299393 A1 discloses a device for use in a body lumen comprising: a microcatheter for delivery of the device, a tubing compartment, a central wire and an engaging compartment, the tubing compartment comprising a pusher tubing and a connecting tubing connected to each other wherein the engaging compartment comprises a proximal engaging element comprising a plurality of wires and being open at a distal end thereof.

### SUMMARY OF THE INVENTION

According to the invention, a device for use in a body lumen is provided. The device comprising:
a microcatheter for delivery of the device;
a tubing compartment;
a central wire; and
an engaging compartment, wherein
the tubing compartment comprises a pusher tubing and a connecting tubing connected to each other; and
the engaging compartment comprises:
   a proximal engaging element comprising a plurality of wires or made from a tubing by a laser cutting technique, said proximal engaging element being open at a distal end thereof, the distal end of said proximal engaging element being bent inward so that tips of the proximal engaging element are atraumatic to an inner wall of the body lumen; and
   a distal engaging element comprising a plurality of wires or struts, said distal engaging element being self-expandable and a proximal end thereof placed between an outer connector and the central wire,
wherein a portion of the occlusion can be captured between the proximal end of the distal engaging element and the distal end of the proximal engaging element when the distance between the distal engaging element and the proximal engaging element is shortened;
wherein the proximal end of the distal engaging element is fixed at the distal end of the central wire, said central wire extending to a proximal end of the device, whereby the position of the distal engaging element in the body lumen is controlled by movement of the central wire; and
wherein the occlusion may be engaged:
   (a) between of the distal engaging element and the wall of the body lumen; and
   (b) between the proximal engaging element and the distal engaging element;
   (c) between the tubing compartment and the wall of the body lumen,
   (d) between the microcatheter and the proximal engaging element,
   (e) between the proximal engaging element and the wall of the body lumen, and
   (f) between a tip of the microcatheter and the same time between the proximal engaging element and the distal engaging element. Preferred embodiments of the invention are defined by the dependent claims.

Preferably, in the device of the present invention the proximal engaging element is fixed with the tubing compartment extending to a proximal end of the device, thereby a location of the proximal engaging element in the body lumen is controlled by movement of the tubing compartment.

Also preferably, in the device of the present invention the tubing compartment is made from one piece of tubing with variable stiffness such that a distal end of the tubing compartment is soft and flexible so the device can pass tortuous anatomy while a proximal end of the tubing compartment is stiff to enhance pushability of the device.

Preferably, in the device of the present invention the central wire comprises a wire, a cable, or a braid.

Also preferably, in the device of the present invention the proximal engaging element comprises a distal end facing the distal engaging element, said distal end of the proximal engaging element being rounded, or smoothed.

Preferably, in the device of the present invention the distal end of the proximal engaging element is configured not to be in direct contact with a surface of the body lumen.

Also preferably, in the device of the present invention the positions of the distal engaging element and the proximal engaging element are configured to lock such that the movement of the two engaging elements after being locked is synchronized and their introduction into a microcatheter is in succession.

Preferably, in the device of the present invention the proximal engaging element and the distal engaging element are configured to be independently controlled.

Also preferably, in the device of the present invention the distal engaging element has an open-end at a distal end.

Preferably, in the device of the present invention the distal engaging element, the outer connector, and the central wire are connected by joining media.

Also preferably, in the device of the present invention the joint media comprises a clip, clasp or fastener.

Preferably, the device of the present invention further comprises a proximal engaging element connector. More preferably, the proximal engaging element connector comprises joining media and an outer proximal element connector. Even more preferably, a proximal end of the proximal engaging element is placed between the outer proximal element connector and a tip of the connecting tubing.

Preferably, the device of the present invention comprises markers for identifying the location of the device.

Also preferably, in the device of the present invention the proximal engaging element is configured to be pushed forward to engage the occlusion with the distal engaging element.

### BRIEF DESCRIPTION OF THE DRAWINGS

Figure 1 shows a non-limiting illustrative example of a device according to some embodiments.
Figure 2A-F shows another non-limiting illustrative example of a device according to some embodiments, particularly when it is located in a body lumen, and illustrates some non-limiting examples of a mechanism to remove an occlusion/clot from and/or expands a blood vessel according to some embodiments of the invention.
Figure 3A-D shows still another non-limiting illustrative example of a device according to some embodiments.
Figure 4A-C shows still another non-limiting illustrative example of a device according to some embodiments.
Figure 5A-C shows still another non-limiting illustrative device according to some embodiments.
Figure 6A-C shows still another non-limiting illustrative example of a device according to some embodiments.
Figure 7A-D shows still another non-limiting illustrative example of a device according to some embodiments.
Figure 8A-D shows still another non-limiting illustrative example of a device according to some embodiments.
Figure 9A-E shows still another non-limiting illustrative example of a device according to some embodiments.
Figure 10A-D shows still another non-limiting illustrative example of a device according to some embodiments.
Figure 11 shows still another non-limiting illustrative example of a device according to some embodiments.
Figure 12A-D shows still another non-limiting illustrative example of a device according to some embodiments.
Figure 13 shows still another non-limiting illustrative example of a device according to some embodiments.
Figure 14A-D shows a non-limiting illustrative example of a process of making a device according to some embodiments.
Figure 15 illustrate non-limiting illustrative examples of an apparatus comprising a device according to some embodiments.
Figure 16 shows still another non-limiting illustrative example of a device according to some embodiments.
Figure 17A-B shows still another non-limiting illustrative example of a device according to some embodiments.
Figures 18 illustrate non-limiting illustrative examples of an apparatus comprising a device according to some embodiments.
Figure 19A-H illustrates some non-limiting illustrative examples of a way that blood occlusion/clot is removed or the vessel is expanded according to some embodiments.
Figure 20A, A', B and C shows a non-limiting illustrative embodiment of a device. Figure 20A shows an open status of the device, Figure 20B shows a closed status of the device (between the distal and proximal engaging elements) and Figure 20C shows a device with closed ended distal tip of the distal engaging element. Figure 20A' shows an embodiment where the proximal engaging element is made from a tubing.
Figure 21A-C shows a non-limiting illustrative embodiment of a method for removing an occlusion or part of an occlusion from a body lumen.
Figure 22A-D shows another non-limiting illustrative embodiment of a method for removing an occlusion or part of an occlusion from a body lumen.
Figure 23A-F shows some other non-limiting illustrative embodiments of a method for removing an occlusion from a body lumen.
Figure 24 shows a non-limiting illustrative embodiment of a system.
Figure 25 shows another non-limiting illustrative embodiment of a system.
Figure 26A-B shows another non-limiting illustrative embodiment of a device. Figure 26A shows an open status of the device and Figure 26B shows a closed status of the device.
Figure 27A-C shows a non-limiting illustrative embodiment of a method for removing an occlusion or part of an occlusion from a body lumen.
Figure 28 shows a still another non-limiting illustrative embodiment of a system.
Figure 29 shows a still another non-limiting illustrative embodiment of a system.
Figure 30A-B shows still a non-limiting illustrative embodiment of a device according to the invention. Figure 30A shows an open status of the device and Figure 30B shows a closed status of the device.
Figure 31A-C shows a still another non-limiting illustrative embodiment of a method for removing an occlusion from a body lumen.
Figure 32A-B shows still another non-limiting illustrative embodiment of a device. Figure 32A shows an open status of the device and Figure 32B shows a closed status of the device where an occlusion is engaged with the device.
Figure 33A-B shows still another non-limiting illustrative embodiment of a device. Figure 33A shows an open status of the device and Figure 33B shows a closed status of the device where an occlusion is engaged with the device.
Figure 34A-B shows still another non-limiting illustrative embodiment of a device. Figure 34A shows an open status of the device and Figure 34B shows a closed status of the device where an occlusion is engaged with the device.
Figure 35 shows still another non-limiting illustrative embodiment of a device. Especially, the figure illustrates an alternative embodiment of the proximal portion a device.
Figure 36A-C shows still another non-limiting illustrative embodiment of a device. Especially, the figure illustrates an embodiment where a microcatheter comprises a tip that is transformable into a different shape.
Figure 37A-D shows a non-limiting illustrative embodiment of a method for removing an occlusion from a body lumen, especially using a device illustrated in Figure 36.
Figure 38A-B shows another non-limiting illustrative embodiment of a device, where a microcatheter comprises a transformable tip at about its distal end.
Figure 39A-B shows a still another non-limiting illustrative embodiment of a device, where a microcatheter comprises a transformable tip at about its distal end.
Figure 40A-B shows a still another non-limiting illustrative embodiment of a device, where a microcatheter comprises a transformable tip at about its distal end.
Figure 41A-C shows still another non-limiting illustrative embodiment of a device, where a microcatheter comprises a transformable tip at about its distal end.
Figure 42A-B shows still another non-limiting illustrative embodiment of a device, where a microcatheter comprises a transformable tip at about its distal end.
Figure 43A-B shows still another non-limiting illustrative embodiment of a device, where a microcatheter comprises a transformable tip at about its distal end.
Figure 44A-B shows still another non-limiting illustrative embodiment of a device, where a microcatheter comprises a transformable tip at about its distal end.
Figure 45A-B shows still another non-limiting illustrative embodiment of a device, where a microcatheter comprises a transformable tip at about its distal end.

### <Reference numerals for designating main components in the drawings>

- 1:: Body lumen surface
- 5:: Guide wire
- 10:: Control element/Central wire
- 20:: Pusher tubing
- 21:: Inner pusher tubing
- 22:: Middle pusher tubing
- 23:: Outer pusher tubing
- 24:: Distal pusher tubing
- 25:: Proximal pusher tubing
- 26:: Introducer sheath
- 30:: Microcatheter
- 31:: Connecting tubing
- 35:: Microcatheter hub
- 40:: Expandable compartment
- 41:: Proximal element connector
- 42:: Proximal element joining media
- 43:: Outer proximal element connector
- 50:: Luminal surface
- 60:: Occlusion/Clot
- 65:: Proximal engaging element
- 70:: Proximal element marker
- 80:: Distal element connector
- 81:: Distal element joining media
- 82:: Outer distal element connector
- 90:: Distal engaging element
- 100:: Distal element marker
- 120:: Distal component control handle
- 130:: Joint
- 140:: Distal connector of proximal component
- 150:: Connecting wire of proximal component
- 160:: Segment connector of proximal component
- 170:: Inflatable or expandable element
- 180:: Injection channel
- 190:: Syringe
- 195:: Injection liquid
- 200:: Transformable microcatheter distal tip
- 210:: Outer sheath
- 220:: Wire
- 230:: Channel
- 410:: Reconfigurable element
- 420:: Supportive element
- 425:: Enclosing element
- 430:: Connector
- 431:: Outer connector
- 432:: Inner connector
- 440:: Markers
- 450:: Distal end connector
- 451:: Supporting element distal connector
- 452:: Supporting element outer distal connector
- 453:: Supporting element inner distal connector
- 455:: Connecter of proximal expendable structure
- 460:: Proximal end connector
- 461:: Outer proximal end connector
- 462:: Inner proximal end connector
- 463:: Connector joining media (adhesive, solder etc.)
- 470:: Adjustment tube
- 471:: sliding tube
- 475:: long inner tube
- 480:: Plateau position
- 490:: Control element handle tubing
- 520:: Connecting wire/stretch resistance wire
- 540:: Coil
- 550:: Distal expandable structure/Distal structure
- 560:: Proximal expandable structure/Proximal structure
- 570:: Joining media
- 580:: Pusher tubing connecting points
- 495:: Distal flexible coil

### DETAILED DESCRIPTION OF THE EMBODIMENTS

The present disclosure is generally related to a device used in a body lumen, such as a blood vessel. The device may be positioned in the body lumen to dilate the lumen and/or remove an occlusion/clot from the lumen. While the device is in the portion of the body lumen that is in need of treatment, an operator can maneuver the device to expand the lumen and/or engage the occlusion/clot.

The device is configured to treat conditions related to an ischemic stroke by removing an occlusion/clot from a blood vessel and/or reopen a blood vessel with some underlying stenosis to resume blood flow therein.

Non-limiting examples of blood vessels may include, an artery, a vein and surgically implanted grafts and bypasses serving as components of the circulatory system.

The term "occlusion" or "clot" generally includes any matter partially or completely obstructing a lumen of the blood vessel. The occlusion/clot slows or obstructs a flow (e.g. a stream of blood or any other biological fluid) running through the lumen. Examples of the occlusion/clot may include blood occlusions/clots and atherosclerotic plaques present in the vessel as well as fat or foreign bodies.

The term "stroke" generally includes a condition(s) that is in part caused due to disturbance in blood supply to a brain. The disturbance can be caused by blockage (e.g. ischemic stroke) and/or hemorrhage (e.g. hemorrhagic stroke) of blood. In particular, an ischemic stroke can be caused due to partial or substantial occlusion/clot of blood vessel. Treatment of the ischemic conditions can be applied to blood vessels present in the brain as well as in other tissues such as the heart. Accordingly, the device and method disclosed in this application are not limited to use in any particular organs but can be applied to any blood vessel of the body that needs dilation of the lumen or removal of occlusion/clot to restore blood flow. In addition, the device according to the present invention can be used to treat venous occlusions/clot which may result in other conditions besides ischemia.

The device can be introduced into the blood vessel through a catheter. The "catheter" generally includes a tubular structure that can be inserted into a body lumen, thereby allowing administration of a device and/or chemicals to a body area that needs treatment. The term "microcatheter" may refer to a catheter that is configured to be administered in a relatively small body lumen such as blood vessels.

The term "tubing" generally refers to a tubular shaped object such as a conduit which may comprise a hollow space (e.g. cylindrical) used to hold and/or conduct a contained objected. Tubing can be made of various materials such as metal, plastic, glass, or any combinations thereof.

The term "wire" generally refers to a metallic or non-metallic object drawn out into the form of a thin flexible thread or rod. The length and thickness of a wire can be highly variable from nanometer scales to meter scales.

The term "stent" generally refers to a tubular support placed temporarily or permanently inside a body lumen, e.g. blood vessel, canal, or duct to aid healing or relieve an obstruction. A stent may be made of one or more wires. In some occasions, a stent may be in form of a strut, which generally refers to a rod or bar forming part of a framework and designed to resist compression. In some other occasions, a stent may be in form of wire web or wire mesh.

The sizes of blood vessels vary enormously, from a diameter of about 1 mm (about 0.03 inch) in smaller arteries and veins to about 25 mm (about 1.0 inch) in the aorta. Accordingly, the diameter of the device may range from approximately about 0.25 mm (about 0.01 inch) in collapsed state to about 25 mm (about 1.0 inch) in expanded state.
The present invention provides a device for use in a body lumen comprising:
a microcatheter (30) for delivery of the device;
a tubing compartment (20, 31);
a central wire (10); and
an engaging compartment, wherein
the tubing compartment (20,31) comprises a pusher tubing (20) and a connecting tubing (31) connected to each other; and
the engaging compartment comprises:
   a proximal engaging element (65) comprising a plurality of wires or made from a tubing by a laser cutting technique, said proximal engaging element (65) being open at a distal end thereof, the distal end of said proximal engaging element (65) being bent inward so that tips of the proximal engaging element are atraumatic to an inner wall of the body lumen; and
   a distal engaging element (90) comprising a plurality of wires or struts, said distal engaging element (90) being self-expandable and a proximal end thereof placed between an outer connector (82) and the central wire (10),
wherein a portion of the occlusion (60) can be captured between the proximal end of the distal engaging element (90) and the distal end of the proximal engaging element (65) when the distance between the distal engaging element (90) and the proximal engaging element (65) is shortened;
wherein the proximal end of the distal engaging element (90) is fixed at the distal end of the central wire (10), said central wire (10) extending to a proximal end of the device, whereby the position of the distal engaging element (90) in the body lumen is controlled by movement of the central wire (10); and
wherein the occlusion (60) may be engaged:
   (a) between of the distal engaging element (90) and the wall of the body lumen; and
   (b) between the proximal engaging element (65) and the distal engaging element (90);
   (c) between the tubing compartment (21,30) and the wall of the body lumen, 56
   (d) between the microcatheter (30) and the proximal engaging element (65),
   (e) between the proximal engaging element (65) and the wall of the body lumen, and
   (f) between a tip of the microcatheter (30) and the same time between the proximal engaging element (65) and the distal engaging element (90).

Preferably, in the device of the present invention the proximal engaging element (65) is fixed with the tubing compartment (20, 31) extending to a proximal end of the device, thereby a location of the proximal engaging element (65) in the body lumen is controlled by movement of the tubing compartment (20, 31).

Also preferably, in the device of the present invention the tubing compartment (20, 31) is made from one piece of tubing with variable stiffness such that a distal end of the tubing compartment (20, 31) is soft and flexible so the device can pass tortuous anatomy while a proximal end of the tubing compartment is stiff to enhance pushability of the device.

Preferably, in the device of the present invention the central wire (10) comprises a wire, a cable, or a braid.

Also preferably, in the device of the present invention the proximal engaging element (65) comprises a distal end facing the distal engaging element (90), said distal end of the proximal engaging element (65) being rounded, or smoothed.

Preferably, in the device of the present invention the distal end of the proximal engaging element (65) is configured not to be in direct contact with a surface of the body lumen.

Also preferably, in the device of the present invention the positions of the distal engaging element (90) and the proximal engaging element (65) are configured to lock such that the movement of the two engaging elements after being locked is synchronized and their introduction into a microcatheter (30) is in succession.

Preferably, in the device of the present invention the proximal engaging element (65) and the distal engaging element (90) are configured to be independently controlled.

Also preferably, in the device of the present invention the distal engaging element (90) has an open-end at a distal end.

Preferably, in the device of the present invention the distal engaging element (90), the outer connector (82), and the central wire (10) are connected by joining media.

Also preferably, in the device of the present invention the joint media (81) comprises a clip, clasp or fastener.

Preferably, the device of the present invention further comprises a proximal engaging element connector (41). More preferably, the proximal engaging element connector (41) comprises joining media (42) and an outer proximal element connector (43). Even more preferably, a proximal end of the proximal engaging element (65) is placed between the outer proximal element connector (43) and a tip of the connecting tubing (31).

Preferably, the device of the present invention comprises markers (100) for identifying the location of the device.

Also preferably, in the device of the present invention the proximal engaging element (65) is configured to be pushed forward to engage the occlusion (60) with the distal engaging element (90).

The control element may comprise a wire, braid, or cable and be configured to control a configuration of the expandable compartment. Various materials can be used to manufacture the control element, which may include metal and non-metal materials. Some non-limiting examples of metal materials for the control element may comprise nickel, titanium, stainless steel, cobalt, chrome and any alloys of the foregoing such as Nitinol (NiTi), stainless steel, or Cobalt Chromium alloys. In addition, any polymers or plastics which have desired properties of being the control element can be used for production of the same. Polymers include, but not limited to, Polyimide, PEEK (Polyether ether ketone), Nylon, PTFE (polytetrafluoroethylene), PET (Polyethylene terephthalate), Polypropylene, etc. Polymer coated metal including but not limited to, PTFE coated Stainless Steel, or PTFE coated NiTi can also be used as control element; The control element can also be made of composite materials, such as PTFE or FEP (Fluorinated ethylene propylene) tubing over NiTi or Stainless Steel etc.

The diameter of the control element may range approximately from 3.937x10⁻⁵ mm to 0.00393 mm (0.001 inch to 0.10 inch).

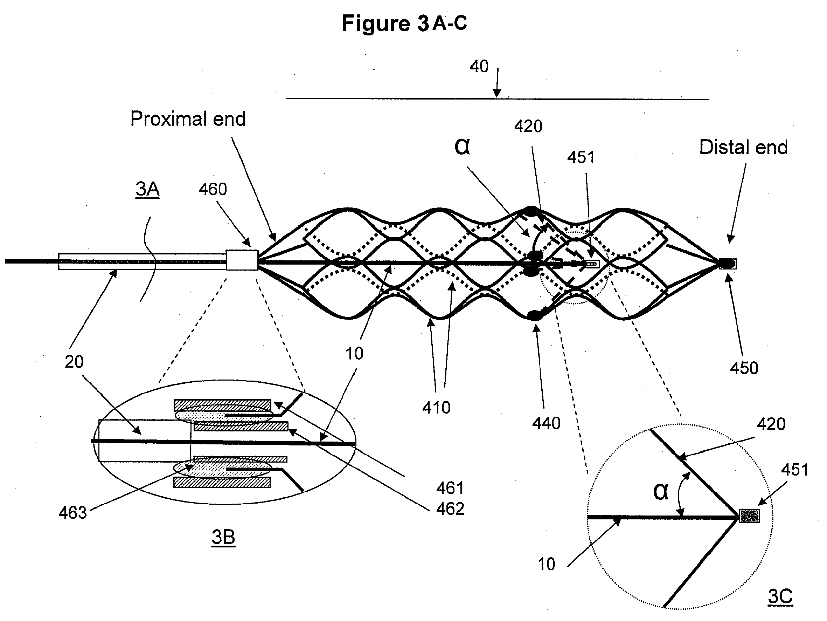

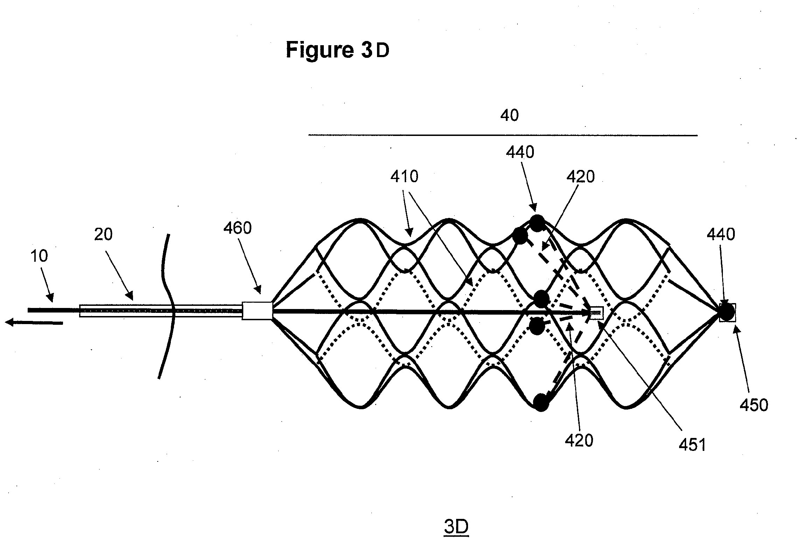

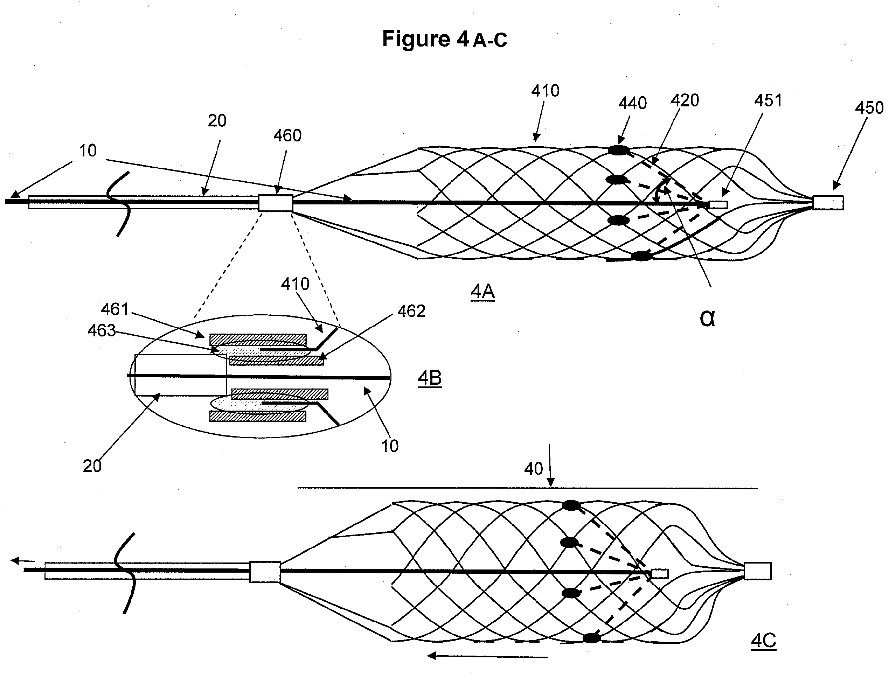

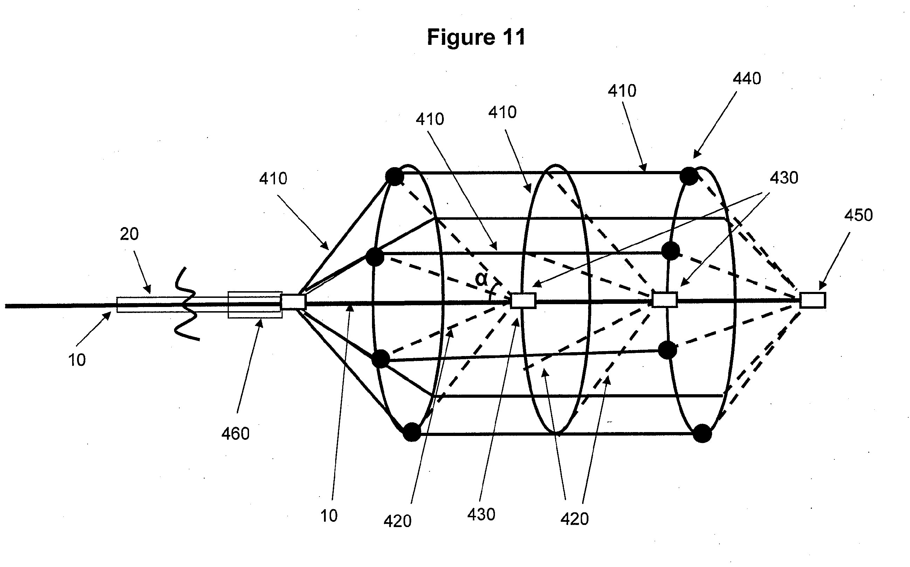

The term "expandable compartment" generally includes a structure that can be inserted into a body lumen to recanalize the blocked vessel or counteract localized flow constriction either by opening the vessel or removing the occlusion/clot. Reconfigurable element is one component to form an expandable compartment. In some embodiments, the reconfigurable element may comprise struts made from tubing or sheet materials (see example in Figure 3). In some other embodiments, the reconfigurable element may comprise a plurality of wires which can be formed into a mesh (see example in Figure 4). In some other embodiments, the plurality of wires of the reconfigurable element may be aligned together and form a tubular shape (see example in Figure 11). The reconfigurable element can be made of metal materials. Some non-limiting examples of such metal materials for the reconfigurable element include nickel-titanium (NiTi) alloy, stainless steel, titanium and its alloys, and cobalt chrome (CoCr) alloys etc. Alternatively, any polymers or plastics which have desired properties of being reconfigurable element can be used as materials of reconfigurable element production. In further alternative examples, the reconfigurable element can be constructed using two or more different materials.

A diameter of the struts used in the reconfigurable element may vary from approximately 12.5 µm to 2500 µm (0.0005 inch to 0.1 inch). In some other embodiments, a diameter of the wire used in the reconfigurable element may vary from approximately 12.5 µm to 2500 µm (0.0005 inch to 0.1 inch). The reconfigurable elements are in general flexible and with elastic or super-elastic property. Thus the reconfigurable element's configurations can be reconfigurable. The reconfigurable element, typically comprise at least three different configurations which are referred to a "collapsed (i.e. axially extended, folded or closed)" configuration, "relaxed (i.e. unfolded or open)" configuration, and an "expanded (i.e. radially extended or radially expanded)" configuration. The complete collapsed configuration of the reconfigurable element generally represents a status in which the outer radius of the reconfigurable element becomes minimized while its axial length is maximized. When the device is in its introducer sheath or in a microcatheter, the reconfigurable element is in its collapsed configuration. When the reconfigurable element is pushed out of microcatheter or introducer sheath and if there is no compressive force, i.e. without any constraint, the reconfigurable element is in its relaxed status. The expanded configuration of the reconfigurable element generally represents a status in which the outer radius of the reconfigurable element becomes further expanded. The configurations of the reconfigurable element may be controlled by the control element and supportive element from its collapsed status, or relaxed status to expanded status. The outer diameter may vary as the reconfigurable element's configuration changes and could range from approximately 0.25 mm to 12.5 mm (0.01 inch to 0.5 inches) in the collapsed configuration. The expanded configuration diameter may range from approximately 1.0 mm to 25 mm (0.04 inches to 1.0 inches). An axial length of the reconfigurable element may also vary as its configuration changes. The axial length of the reconfigurable element may be increased as it becomes collapsed. On the contrary, the axial length of the reconfigurable element may be reduced as it becomes more expanded. The axial length of the reconfigurable element could range from approximately 2.5 mm to 75 mm (0.1 inch to 3 inches).

The supportive element comprises a plurality of wires or struts. The plurality of wires or struts of the supportive element may be in a generally linear form or in a generally non-linear form. The supportive element is in a form of wire mesh. The supportive element is in a braid form. In other embodiments, the supportive element is in a meshed tubular form manufactured from a tube though laser-cutting. The supportive element together with the reconfigurable element are in a meshed sheet form, manufactured through laser cutting or photo etching process. The supportive element is generally configured to adjust a configuration of the reconfigurable element, thereby providing delicate control over the extent of the reconfigurable element's radial expansion. Such delicate control mechanism of the device would be beneficial in many aspects. After delivery and release of the device at the selected treatment site, it may appear that the radius and/or radial force of the self-expanding reconfigurable element may be less than that desired for the application. On such occasion, the supportive element may provide further radial force/pressure to the lumen. It may be desired to occasionally increase or decrease the amount of radial force which the device exerts against surrounding tissue or occlusion/clot. In such cases, the configuration of the reconfigurable element can be dynamically controlled to provide a wider range of radial force in the device according to the present disclosures. The device may also reduce or minimize any unnecessary impact or damage to the blood vessel while the device is being delivered, removed and/or operated. When the device is delivered, it may provide undesired pressure and/or impact to the lumen when it is released in the vessel and can expand more than the luminal diameter. In such cases, the reconfigurable element's diameter and radial force can be reduced by movement of the control element and supportive element when necessary. In other cases, when the device is being removed the radial force may be too great and potentially cause injury while being pulled back through the blood vessel. Similarly, the reconfigurable element's diameter and radial force can be reduced by movement of the control element and supportive element.

The engaging component/compartment may comprise a distal engaging element and a proximal engaging element. In some embodiments, the distal engaging element may be associated with the central wire. One or both of the proximal and distal engaging elements may be engaging element(s). The distance between the distal and proximal engaging elements is adjustable. The distance between the proximal and distal engaging elements can be adjusted approximately from 0 to 50 mm in at least some embodimentsThe distance between the proximal and distal engaging elements may be adjusted approximately 0 mm, 5 mm, 10 mm, 15 mm, 20 mm, 25 mm, 30 mm, 35 mm, 40 mm, 45 mm, and 50 mm, and any range there between. The distance between the proximal and distal engaging elements may be adjusted to be more than 50 mm.

The device can be introduced into a blood vessel. The sizes of blood vessels vary enormously, from a diameter of about 1 mm (about 0.03 inch) in smaller arteries and veins to about 25 mm (about 1.0 inch) in larger arteries. Accordingly, the diameter of the device may range from approximately about 0.25 mm (about 0.01 inch) to about 25 mm (about 1.0 inch). Also, the diameter of a single device may vary during the operation as the engaging compartment gets opened (or expanded) or closed (or collapsed).

The device further comprises a central wire. The central wire may pass through the tubing component and move freely there through. The control wire is associated with the engaging compartment. More particularly, the central wire may be associated with the distal engaging element and the proximal engaging element. Association generally refers to any type of connection between two objects. Association includes fixation in that when two objects are associated, movement of one object would be hindered by another object. In other words, once the two objects are associated in a way of fixation, movement of two objects would be synchronized. However, association does not necessarily indicate fixation of one object to another. Accordingly, when two objects are associated but not in a state of fixation, movement of one object with respect to the other object would not be hindered. Therefore, the distal engaging element and the proximal engaging element, both of which are associated with the central wire, may mover freely along the central wire, in at least some embodiments.

The central wire is fixed or joined with the distal engaging element. In some occasions, the proximal end of the distal engaging element may be joined to the distal end of the central wire. The association (i.e. connection) between the central wire and the distal engaging element may be done via various ways such as welding, gluing, or clipping. In some embodiments, the joint between the central wire and the distal engaging element is covered by a distal element connector. Alternatively, no coverage would be provided to surround the connected control wire and the distal engaging element.

The central wire may comprise or in be in the form of a wire, braid, or cable. Various materials can be used to manufacture the central wire, which may include metal and non-metal materials. Some non-limiting examples of metal materials for the central wire may comprise nickel, titanium, stainless steel, cobalt, chrome and any alloys of the foregoing such as Nitinol (NiTi), or Cobalt Chromium alloys. In addition, any polymers or plastics which have desired properties of being the central wire can be used for production of the same. Polymers include, but not limited to, Polyimide, PEEK (Polyether ether ketone), Nylon, PTFE (polytetrafluoroethylene), PET (Polyethylene terephthalate), Polypropylene, etc. Polymer coated metal including but not limited to, PTFE coated Stainless Steel, or PTFE coated NiTi can also be used as a central wire. Also hydrophilic coating would be applicable. Such coating can be applicable in part to reduce friction between the central wire and the tubing compartment(s). The central wire can also be made of composite materials, such as PTFE or FEP (Fluorinated ethylene propylene) tubing over NiTi wire, or PTFE or FEP tubing over Stainless Steel etc. The diameter of the central wire may range approximately from 3.93x10⁻⁵ mm to 0.00984 mm (0.001 inch to 0.25 inch). The diameter of the central wire may be about 3.93x10⁻⁵, 7.87x10⁻⁵, 0.000118, 0.000157, 0.000196, 0.000236, 0.000275, 0.000314, 0.000354, 0.000433, 0.000472, 0.000511, 0.000551, 0.000590, 0.000629, 0.000669, 0.000708, 0.000748, 0.000787, 0.000826, 0.000866, 0.000905, 0.000944 and 0.000984 mm (0.001, 0.002, 0.003, 0.004, 0.005, 0.006, 0.007, 0.008, 0.009, 0.011, 0.012, 0.013, 0.014, 0.015, 0.016, 0.017, 0.018, 0.019, 0.020, 0.021, 0.022, 0.023, 0.024 and 0.025 inch). Alternatively, the diameter of the central wire may be more than 0.000984 mm (0.025 inch).

The term "engaging compartment" generally includes a structure that can be compressed into small diameter and inserted into a body lumen and, upon releasing compression, expands to a larger diameter to recanalize the blocked vessel or counteract localized flow constriction either by opening the vessel or removing at least part of the occlusion. The engaging compartment may comprise a distal engaging element and a proximal engaging element. The distal and proximal engaging elements can be a braid structure in at least some embodiments. They can also be made through laser cut hypo-tubes, or photo etched sheet materials. Heat treatment may be needed to set them into the desired shape, e.g. cone shape or cylinder shapes.

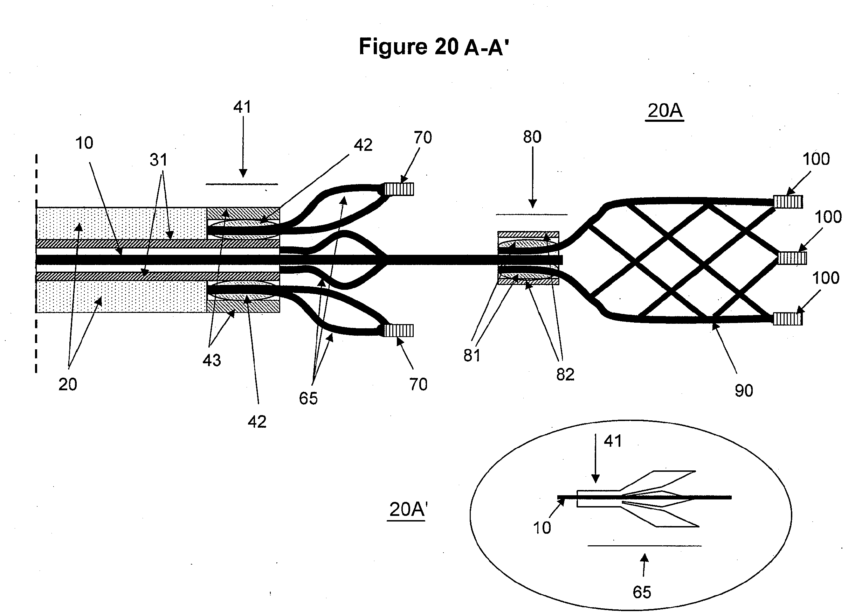

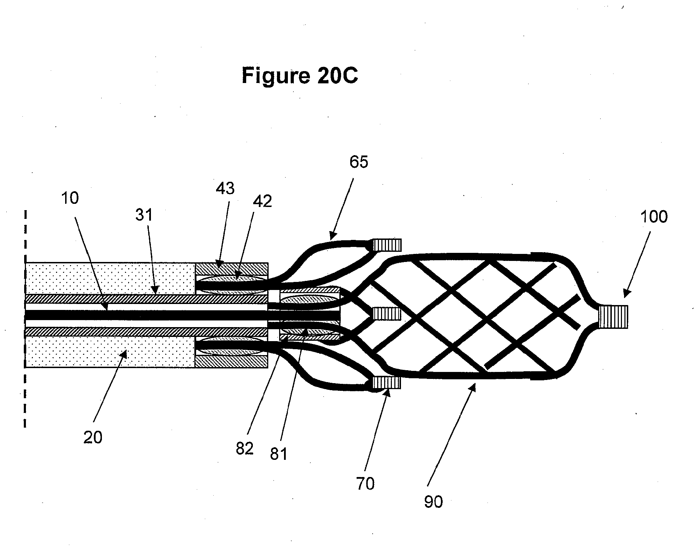

The engaging elements may comprise a stent made from tubing or sheet materials. In some other embodiments, the engaging elements may comprise a plurality of wires which can be formed into a mesh. In some occasions, the distal end of the distal engaging element may be closed as seen in Figure 20C. Alternatively, the distal end of the distal engaging element may stay opened as seen in Figure 20A.

The distal engaging element can be made of metal materials. Some non-limiting examples of such metal materials for the distal engaging element include nickel-titanium (NiTi) alloy, stainless steel, titanium and its alloys, and cobalt chrome (CoCr) alloys. Alternatively, any polymers or plastics which have desired properties for a distal engaging element can be used. In some embodiments, the distal engaging element is made of flexible material(s). In further alternative examples, the distal engaging element can be constructed using two or more different materials, such as polymer coated metal materials.

The diameter of the distal engaging element may vary from approximately 1 to 8 mm at its expanded state. The diameter of the distal engaging element at its expanded state may be approximately 1 mm, 2 mm, 3 mm, 4 mm, 5 mm, 6 mm, 7 mm, and 8 mm or any range therebetween. The length of the distal engaging element may vary from approximately 10 to 40 mm. The length of the distal engaging element may be approximately 10 mm, 15 mm, 20 mm, 25 mm, 30 mm, 35 mm, and 40 mm or any range therebetween. Further, the length of the distal engaging element may be more than 40 mm.

The distal engaging elements are in general flexible and with elastic or super-elastic property. Thus the distal engaging element may be varied in its shape. The distal engaging element, typically comprise at least two different configurations which are referred to a "collapsed (i.e. folded or closed)" configuration and a "relaxed (i.e. unfolded or open)" configuration. The collapsed configuration of the distal engaging element generally represents a status in which the outer radius of the distal engaging element becomes minimized. When the distal engaging element is in a microcatheter, the distal engaging element is in its collapsed configuration. When the distal engaging element is pushed out of microcatheter and if there is no compressive force constraining it, the distal engaging element is in its relaxed status. In some embodiments, the distal engaging element may comprise a stent that is self-expandable. Accordingly, once the stent is pushed out of the microcatheter or the microcatheter is withdrawn leaving the stent distal to the microcatheter, the stent is not constrained and it will be expanded on its own. Due to its flexible property, the distal engaging element may be easily placed into the microcatheter by gently pulling or pushing the same toward the microcatheter.

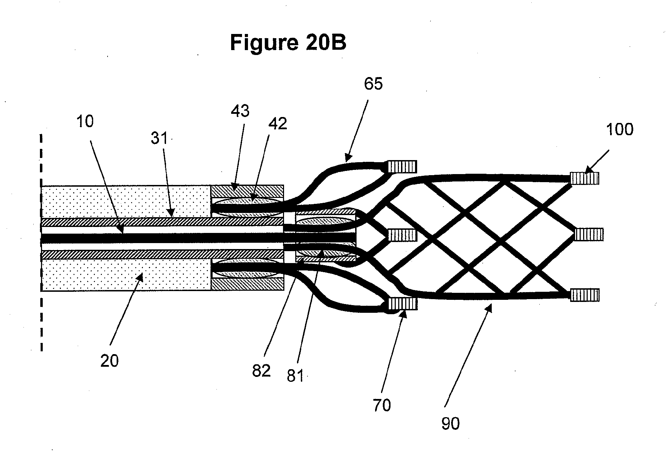

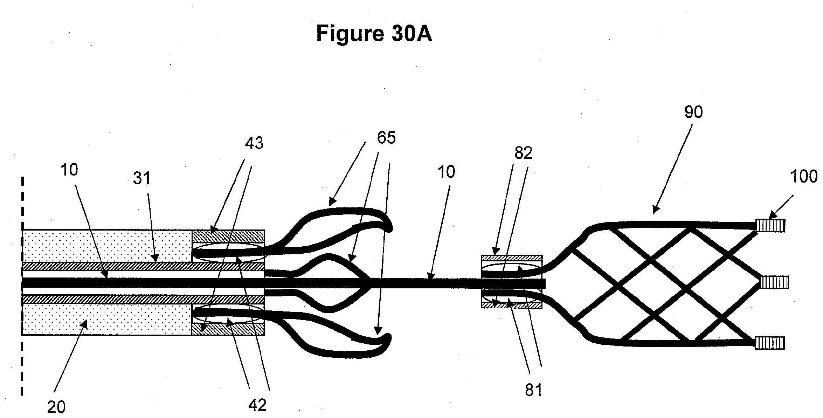

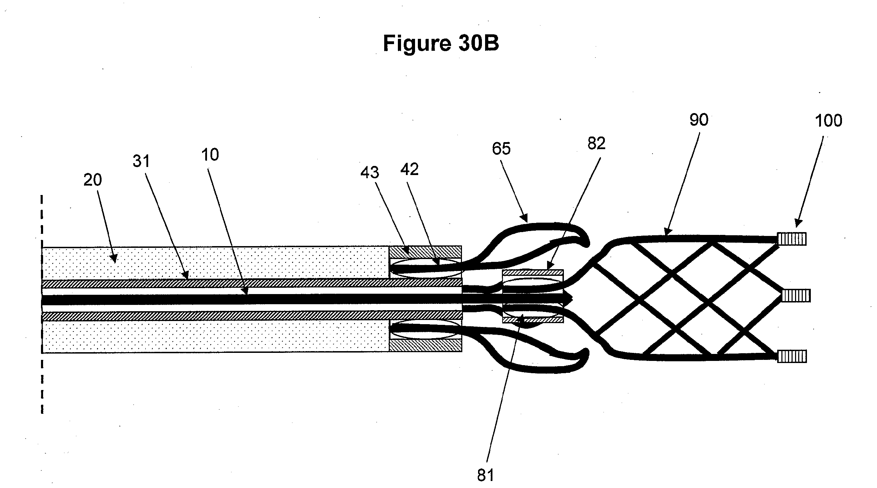

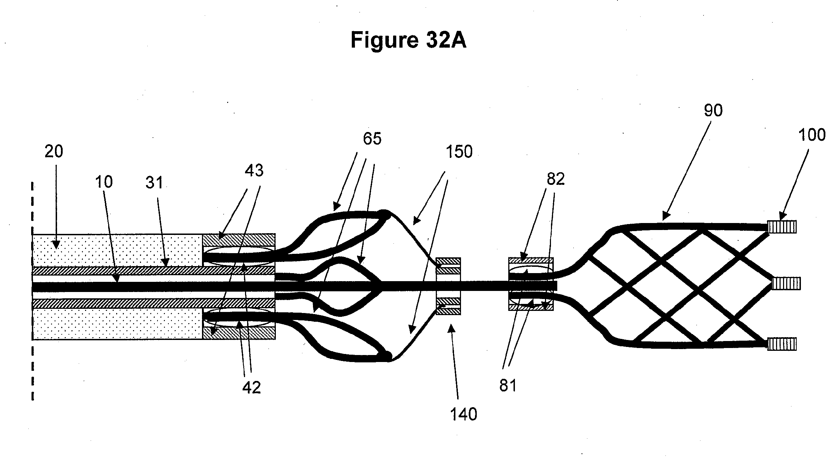

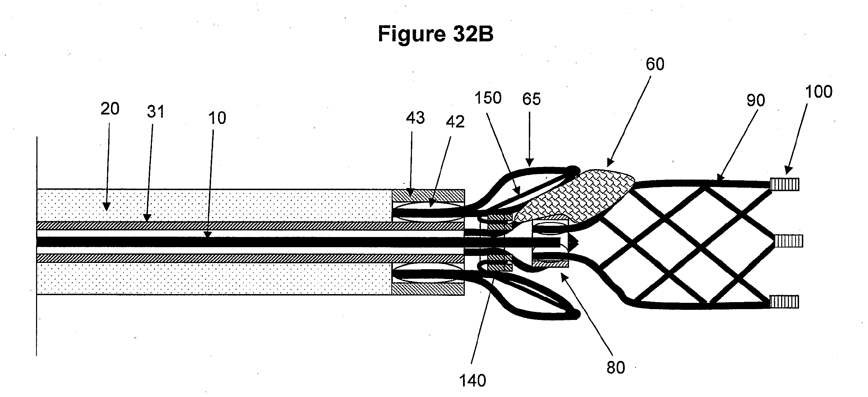

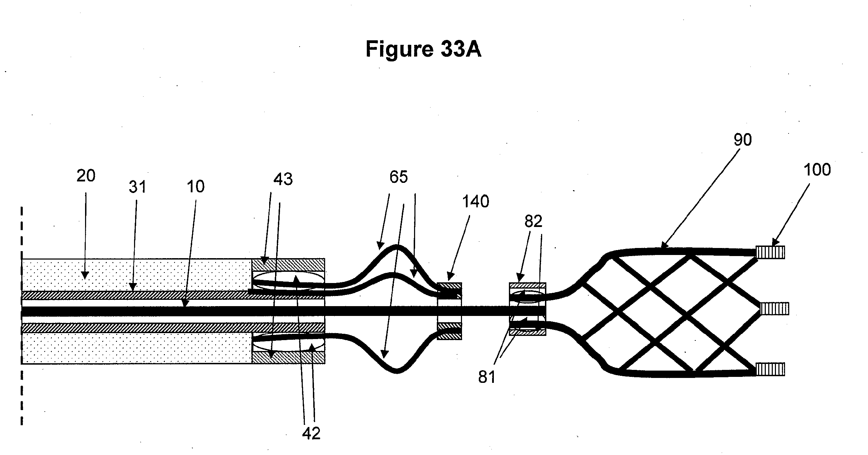

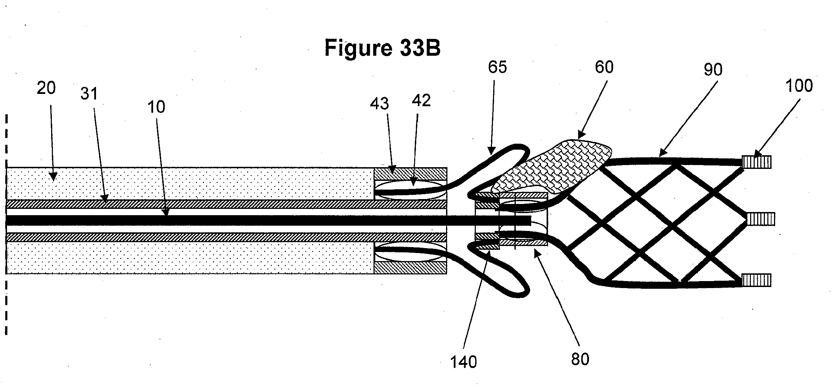

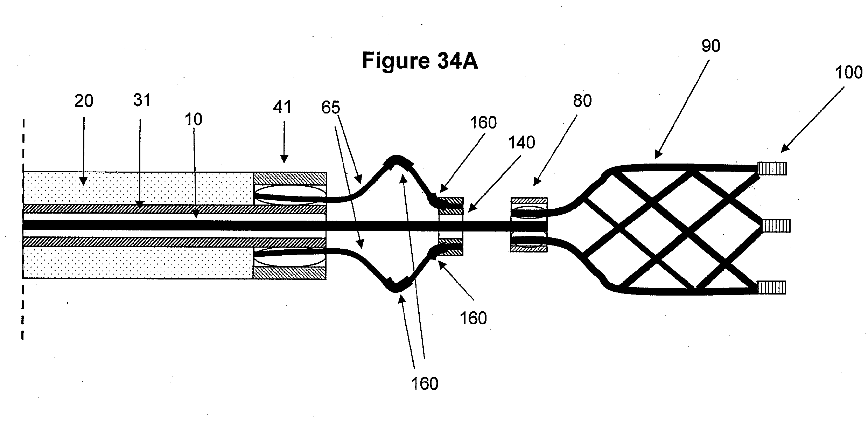

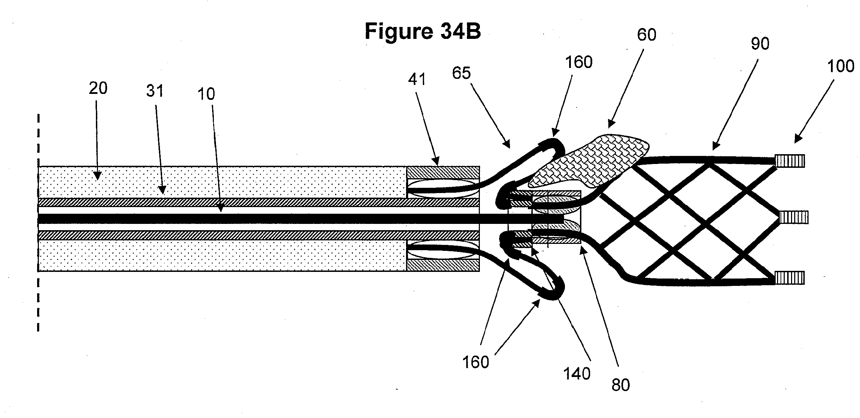

The proximal engaging element may comprise one or more wires made from tubing or sheet material(s). In some other embodiments, the proximal engaging element may comprise one or more wires which can be formed into a basket-like form, e.g. as shown in Figure 20. Alternatively, the proximal engaging element can be manufactured in many shapes or forms. For example, the distal end of the proximal engaging element, which may face toward the body lumen surface, may be modified in order to reduce any damage to the surface of the body lumen. Accordingly, the distal end of the proximal engaging element has been substantially smoothened so that even in direct contact with the proximal engaging element the body lumen would remain largely undamaged. Also, the distal end of the proximal engaging element would be curved, e.g. as shown in Figure 30, so that the pointed end would not be in direct contact with the body surface. Further, the shape or form of a proximal engaging element can be varied during the operation. Therefore, the distal end, more particularly, the distal tip of the proximal engaging element may be configured to be removed during the operation. For instance, as illustrated in Figures 32-34, the distal tip of the proximal engaging element is connected to a connector directly (e.g. Figures 33 and 34) or indirectly (e.g. via a connecting wire as illustrated in Figure 32). With these configurations, the distal tip of the proximal engaging element would be moved away from the body lumen via movement of the connector. More particularly, when the proximal connector of the distal element together with a clot moves proximally, it may pull the distal tip of the proximal engaging element proximally and fold the proximal element into the desired basket shape with the tip being round shape, or atramatic. Therefore, the risk of damaging the body lumen by directly contacting the body lumen surface (e.g. blood vessel) would be substantially reduced, in at least some embodiments. These tip features also makes it possible for the proximal engaging element to be pushed forward in the lumen when needed and not injure the surface of the lumen.

The proximal engaging element is configured to significantly improve clot engagement and retrieval efficiency. For example, as demonstrated in Figure 3, an occlusion may be disposed between the proximal and distal engaging elements when using the device according to some embodiments. This design has an improved ability to engage an occlusion or clot more firmly by holding it with two separate engaging elements.

The proximal engaging element can be made of metal materials. Some non-limiting examples of such metal materials for the proximal engaging element include nickel-titanium (NiTi) alloy, stainless steel, titanium and its alloys, and cobalt chrome (CoCr) alloys. Alternatively, any polymers or plastics which have desired properties for a proximal engaging element can be used. In some embodiments, the proximal engaging element is made of flexible material(s). In further alternative examples, the proximal engaging element can be constructed using two or more different materials, e.g. as illustrated in Figure 34.

A diameter of the proximal engaging element may vary from approximately 1 to 8 mm at its expanded state. The diameter of the proximal engaging element at its expanded state may be approximately 1 mm, 2 mm, 3 mm, 4 mm, 5 mm, 6 mm, 7 mm, and 8 mm or any range therebetween. The length of the proximal engaging element may vary from approximately 2 to 40mm. The length of the proximal engaging element may be approximately 2 mm, 5 mm, 7 mm, 10 mm, 12 mm, 15 mm, 17 mm, 20 mm, 22 mm, 25 mm, 27 mm, 30 mm, 32 mm 35mm, 37 mm, 40 mm or any range therebetween. Further, the length of the proximal engaging element may be more than 40 mm.

The proximal engaging elements are in general flexible and with elastic or super-elastic properties. Thus the proximal engaging element may be varied in its shape. The proximal engaging element, typically comprise at least two different configurations which are referred to a "collapsed (i.e. folded or closed)" configuration, and a "relaxed (i.e. unfolded or open)" configuration. The collapsed configuration of the proximal engaging element generally represents a status in which the outer radius of the proximal engaging element becomes minimized. When the proximal engaging element is in a microcatheter, the proximal engaging element is in its collapsed configuration. When the proximal engaging element is out of microcatheter and is unconstrained, the proximal engaging element is in its relaxed status. In some embodiments, the proximal engaging element is made of elastic or super-elastic material, and thus is self-expandable. Due to its flexible property, the proximal engaging element may be easily placed into the microcatheter by gently pulling or pushing the same toward the microcatheter. In further alternative embodiments, the proximal engaging element may have more than two different statuses. For example, as demonstrated in Figures 32-34, the proximal engaging element may be configured to change its shape during the operation, and resultantly there would many different statuses between the complete collapsed and the complete relaxed statuses.

One or more markers may be added to the device. Such markers may include radiopaque materials which help monitor the position and or movement of the device in the body. Some non-limiting examples of radiopaque markers may comprise gold, gold alloys, CoCr alloy, platinum, or platinum alloys. Marker(s) can also be in form of radiopaque coating. The markers may be added anywhere in the device. One or more markers may be added at the distal engaging element so that a location of the distal engaging element in the body would be determined. One or more markers may be added at the proximal engaging element so that a location of the proximal engaging element in the body would be determined. Both of the distal and proximal engaging elements contain markers. The markers in each of the distal and proximal engaging elements may be the same or different materials. Alternatively, one or more markers may be added to the central wire and/or the tubing compartment. The markers may be approximately 0.10 to 4 mm long, and the diameter is approximately 3.937x10⁻⁵ to 0.00118 mm (0.001 to 0.030 inch). However, any variations in any dimensions (e.g. length, diameter, size, and mass) and in shapes of markers are suitable.

The device may comprise tubing compartments. Tubing compartments may comprise a plurality of tubing elements. Such tubing elements may include a pusher tubing and a connecting tubing. The pusher tubing may further comprise an inner pusher tubing, a middle pusher tubing, an outer pusher tubing in at least some embodiments. Also in alternative embodiments, the pusher tubing may comprise a distal pusher tubing and a proximal pusher tubing. Various materials can be used to manufacture the tubing elements, which may include metal and non-metal materials. In some embodiments, the distal pusher tubing and/or an outer pusher tubing can be made from lubricious and flexible polymers such as PTFE or PET. The middle and proximal pusher tubing can be made from Nitinol super-elastic material, stainless steels, CoCr alloys, titanium alloys, or polymers (such as Polyimide, PEEK, etc.). One or more of the tubing elements can also be coated with lubricious material, such as PTFE coating, hydrophilic coating etc. The tubing elements can also be made of composite materials, such as PTFE or FEP (Fluorinated ethylene propylene) tubing over Nitinol wire, or PTFE or FEP tubing over Stainless Steel etc.

The central wire can be in the form of a wire, braid, or tubing. Some non-limiting examples of metal materials for the central wire may comprise nickel, titanium, stainless steel, cobalt, chrome and any alloys of the foregoing such as Nitinol (NiTi), or Cobalt Chromium alloys. In addition, any polymers or plastics which have desired properties of being the central wire can be used for production of the same. Polymers include, but not limited to, Polyimide, PEEK (Polyether ether ketone), Nylon, PTFE (polytetrafluoroethylene), PET (Polyethylene terephthalate), Polypropylene, etc. Polymer coated metal including but not limited to, PTFE coated Stainless Steel, or PTFE coated NiTi can also be used as a central wire. Also a hydrophilic coating can be applied.

The diameter of the pusher tubing and the connecting tubing may be approximately 3.937x10⁻⁵ to 0.00196 mm (0.001 to 0.050 inch). The diameter of the pusher tubing and the connecting tubing may be smaller than 3.937x10⁻⁵ mm or over 0.00196 mm (0.001 inch or over 0.050 inch). The device may comprise a pusher tubing, but not a connecting tubing.

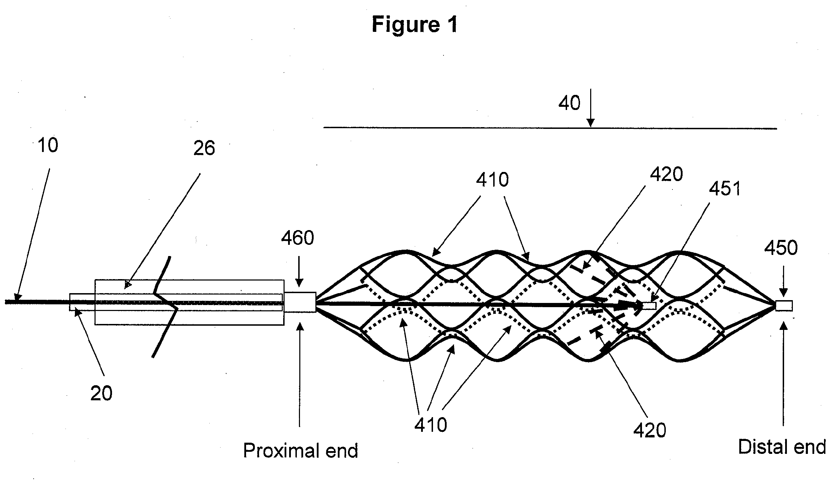

Referring to Figure 1, a device comprising a reconfigurable element (410), a supportive element (420), a control element (10), and a pusher tubing (20) is depicted. The expandable compartment (40) can be present inside the introducer sheath (26).

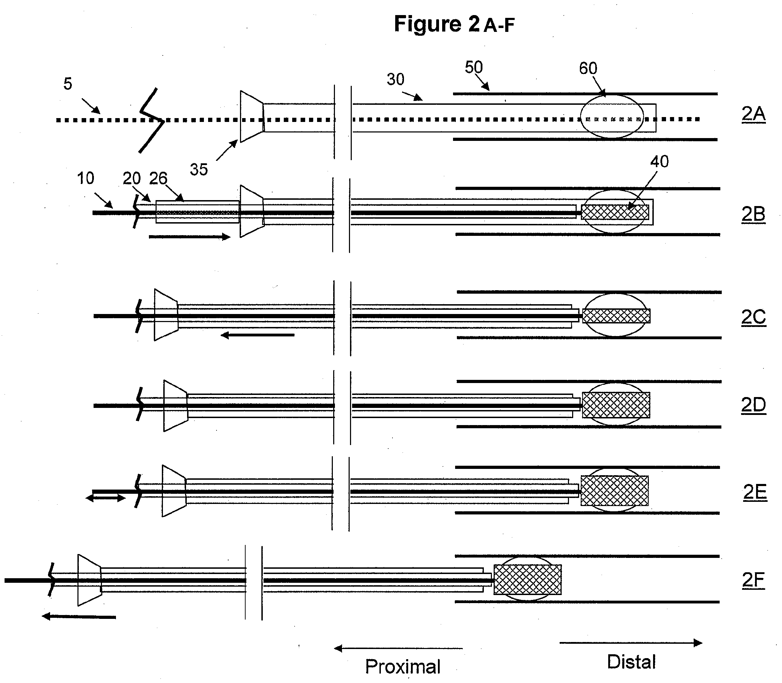

During a clinical procedure, the device can be pushed into a microcatheter (30) via introducer sheath (26) and be further pushed to the lesion site as seen in Figure 2. The reconfigurable element (410) can be connected to a pusher tubing (20), in this case, a thin hollow tube. The supportive element may be associated with the control element. At least part of the control element (10) may be surrounded by a pusher tubing (20) and the control element (10) may freely slide through the pusher tubing (20). Thus, the movements of the control element (10) and the pusher tubing (20) may not be constrained by each other and each can slide freely along the axial axis of the device, as shown in Fig. 3B and Fig. 4B. The microcatheter may be placed at an occlusion/clot location with the help of a guide wire. The pusher tubing can be used to push the device into micro-catheter. The expandable compartment of the device can then be pushed out of the microcatheter during treatment. The radial force and the diameter of the reconfigurable element can be adjusted by pulling or pushing the control element proximally or distally.

Figure 2 illustrates the steps that may need to remove an occlusion/clot from the blood vessel. Accordingly the luminal surface (50) may represent a blood vessel wall. In some embodiments, the device may be delivered to the luminal area (50) in which the occlusion/clot (60) is present. During the delivery, with the help of a guide wire (5), the distal tip of the microcatheter may be navigated to the blocked site and pass through the occlusion/clot (60), as illustrated in Fig. 2A. Angiographic guidance may be used to locate the position of the micro-catheter relative to the vessel and occlusion/clot.

After removing the guide wire (5), the retrieval device can then be introduced into the microcatheter (30). The expendable compartment (40) may be pushed through the microcatheter (30) until the distal end of the expendable compartment (40) reaches to the distal end of the microcatheter (Fig 2B). As depicted in Figure 2C, the microcatheter (30) may be withdrawn slightly by pulling it proximally while the pusher tubing (20) is held stable. The expandable compartment (40) may be exposed to the occlusion/clot (60) and partially opened as shown in Fig. 2D. The operator may adjust the configuration of the reconfigurable element (410), i.e. radial force, diameter, and axial length of the reconfigurable element) to allow the reconfigurable element (410) to break or engage the occlusion/clot (60), disrupt at least part of the occlusion/clot (60) and/or expand the lumen of the vessel. Such adjustment of the reconfigurable element's configuration may be achieved at least by pulling or pushing the control element (10) distally or proximally as seen in Figure 2E. After the occlusion/clot (60) is engaged by the expandable compartment (40), the device, along with the microcatheter (30), may be retrieved from the blood vessel as shown in Figure 2F.

Alternatively, the micro-catheter can be first placed beyond the occlusion/clot and the expandable component can be opened distal to the occlusion/clot. After maneuvering and adjusting the reconfigurable element's diameter and radial force, the device can be pulled proximally, and the occlusion/clot can be entrapped by the expandable compartment and pulled out of vessel.

While the embodiments of Figure 2 illustrate substantial removal of occlusion/clot from the blocking site of the lumen, alternative treatment such as disruption of at least part of the occlusion/clot can be used. The occlusion/clot is often substantially soft and may be broken up with a relatively minor impact. In such cases, the reconfigurable element may in part break up the occlusion/clot into smaller components. The pieces of the occluding material can be collected and removed from the body with the device. If the reconfigurable element's wires or struts cut through the occlusion/clot (e.g. blood clot) while the reconfigurable element expands radially in the blood vessel, portions of the occlusion/clot become contained in, or engage with the expandable compartment as it expands to the full diameter of vessel. By slightly collapsing the reconfigurable element through pulling it partially into the micro-catheter tip, the openings between the wires/struts of the reconfigurable element cells would be made smaller. This may maintain the occlusion/clot in the expandable compartment. The supportive element may also help to maintain the occlusion/clot within the expandable compartment. The whole device containing the portions of the occlusion/clot can then be pulled out of the blood vessel. In further alternative embodiments, the device may dilate/expand the lumen so that the flow can be re-established at the blocking site of the lumen. It is also possible that the occlusion/clot may not be soft enough to allow the device substantially engage the occlusion/clot. In such cases, the configuration of the reconfigurable element may be controlled to engage at least part of the occlusion/clot and mobilize the same. When the reconfigurable element's diameter is further opened, its wires or struts will push against the occlusion/clot. While pulling the proximally, the friction between the reconfigurable element and the occlusion/clot may cause occlusion/clot to become dislodged from the vessel wall and removed.

Figure 3 and 4 shows devices according to some embodiments. The device comprises a pusher tubing (20) which may be connected with proximal end connector (460) of the reconfigurable element (410). The pusher tubing as well as the control element may be substantially long. In some embodiments, the pusher tubing and the control element may long enough for an operator to control the retrieval device from outside a body via the pusher tubing and the control element. In some embodiments, the pusher tubing and the control element may extend about 100 cm, 110 cm, 120 cm, 130 cm, 140 cm, 150 cm, 160 cm, 170 cm, 175 cm, 180 cm, 185 cm, 190 cm, or 200 cm. These wires can be extended over 200 cm, if necessary.

The reconfigurable element (410) may comprise at least two ends, a distal end and a proximal end. The distal end of the reconfigurable element (410) generally refers to an end that may enter the body prior to the proximal end. The proximal end of the reconfigurable element (410) generally refers to an end in which the reconfigurable element (410) is associated with the pusher tubing (20).

In some embodiments, the distal end of the reconfigurable element (410) is closed, which means that the distal ends of the reconfigurable element wires or struts (410) are held together by means of, for example, welding, soldering, or gluing, with or without a connector (430 or 450). In some embodiments, the distal end of the control element (10) and the distal end of the supportive element (420) may be held together with or without connector (451) via a means of, for example, welding, soldering, or gluing etc. Alternatively, a distal end connector (451) may be used to couple the distal tip of control element (10) and the distal tip of supportive element (420). At the proximal end of the reconfigurable element (410), there is an outer proximal end connector (461) and an inner proximal end connector (462), both of which may be tubular structures as seen in Figure 3B-8B. The proximal wire or strut ends of the reconfigurable element (410) may be placed between the inner and outer proximal end connectors (461 and 462) and are fixed in place by means described above. The control element (10) may pass through the lumen of the inner proximal end connector (462) so that it can slide in the proximal end connector (460) freely.

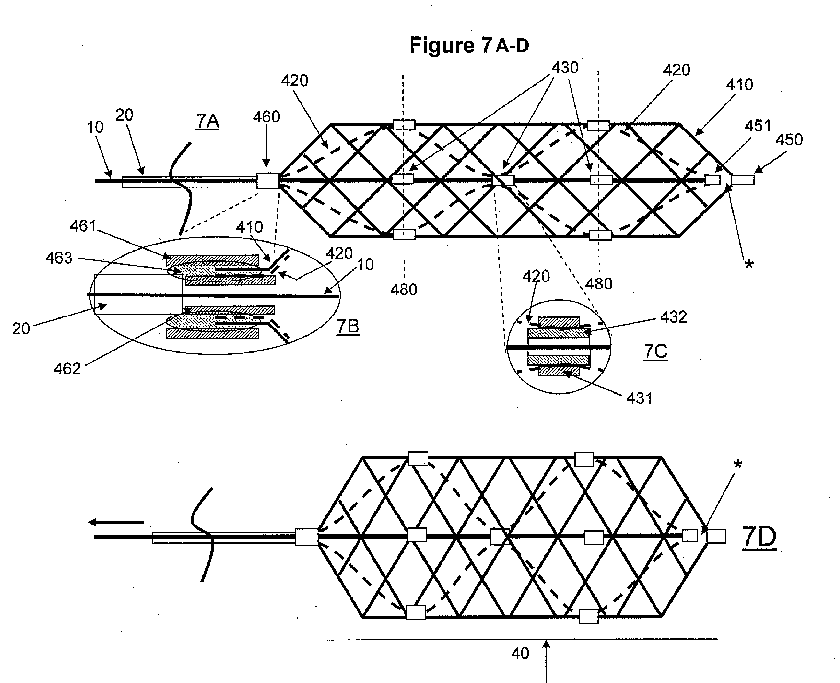

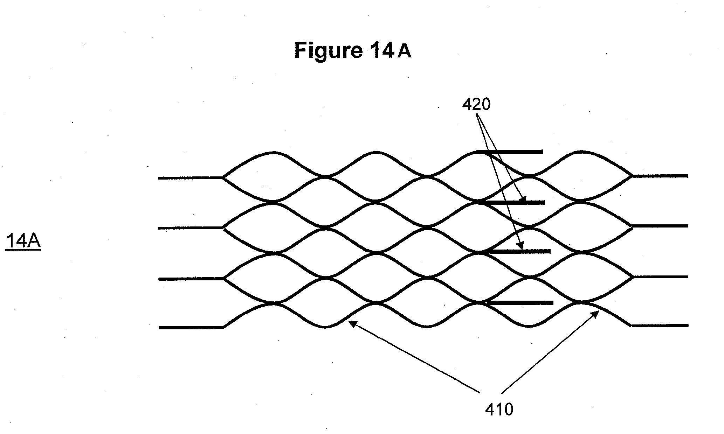

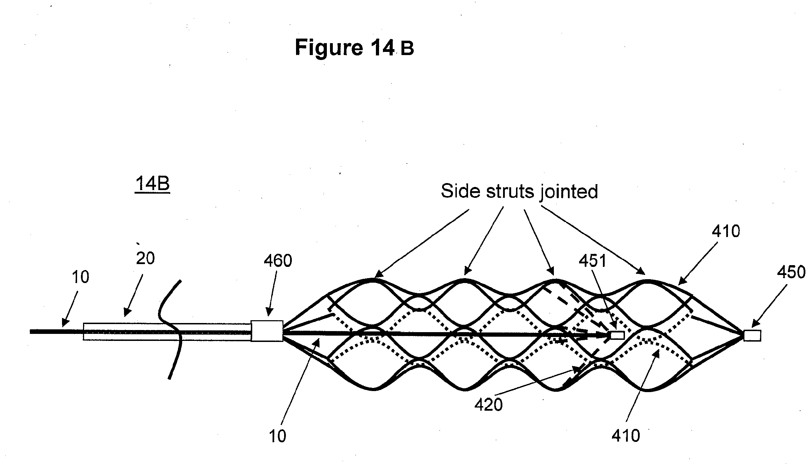

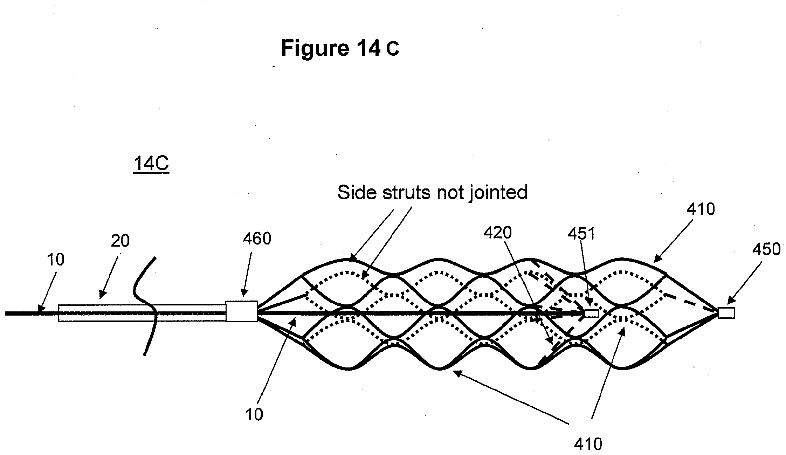

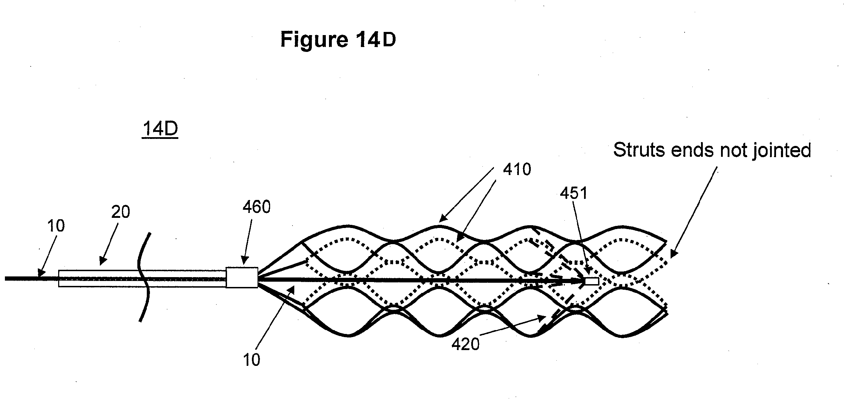

The device further comprises a supportive element (420) which may be associated with the reconfigurable element's wire/strut (410) and the control element (10). The supportive element (420) may be associated with the control element (10) via a connector (430 and/or 451), for example, as seen in Figure 7. In some embodiments, the supportive element (420) is associated with the distal end of the control element (10) in a substantially immobilized manner. Accordingly, as the control element (10) is pulled proximally, the distal end of the supporting element (420) may be pulled proximally. The supportive element (420) may be attached to the reconfigurable element (410) as shown in Figure 3 and Figure 4. In this embodiment the supportive element ends that are attached to the reconfigurable element (410) may move outward causing the reconfigurable element (410) to enlarge (as shown in Figure 3C and Figure 4C). This will increase the radial force of the reconfigurable element. In other embodiments the supportive element (420) is made from the same piece of material as the reconfigurable element (410) as shown in Figure 3 and Figure 14. Thus, joining these two components is not needed. In this embodiment pulling the control element (10) proximally will result in enlargement of angle α (Figure 3C) between the supportive element (420) and the control element (10) causing the reconfigurable element (410) to become enlarged (as shown in Figure 3D). This will increase the radial force of the reconfigurable element.

As further depicted in Figures 3B and 4B, the control element (10) may be present in the lumen of the proximal inner connector (462) and slide freely through the connector (460). In some embodiments, markers (440) may be added to the device, for example, at which the supportive element (420) is associated with the wire/strut (410). Such markers may include radiopaque materials which help visualization/monitoring the position and or movement of the device in the body. Some non-limiting examples of radiopaque markers may comprise gold and/or platinum. The markers may be added at some of the attachment points between the supportive element (420) and any portion of the wire/strut of the reconfigurable element (410), control element, or supportive element (420), if desired. For example, the markers may be applied at about the distal and/or proximal ends of the reconfigurable element or be coated on any part of the wires and/or connectors. The radiopaque property of the device can also be achieved by using CoCr alloy as the reconfigurable element, control element, and, or supportive element.

The supportive element can be constructed in a variety of forms. The particular example shown in Figure 3, Figure 13 and Figure 14 is a build-in structure, i.e. both the supportive element and reconfigurable element are built from one piece of material, either from tubing or flat sheet. Thus additional joining/bonding between them is not needed.

Figure 4 comprises a plurality of supportive element that is in a form of substantially linear wire. Materials used to manufacture supportive elements would be metal or non-metal materials. Some non-limiting materials used for the supportive element include, but not limited to, nickel, titanium, NiTi, stainless steel, cobalt chrome and any alloys of the foregoing. The configuration of the reconfigurable element may be controlled by movement of the control element and the supportive element. After the microcatheter is passed through occlusion/clot, the device may be delivered into the microcatheter. When the device is unsheathed from the microcatheter (*See,* for example, Figure 2), it can be exposed and engage with the occlusion/clot.

In some embodiments, the reconfigurable element may be self-expanding once it is out of the microcatheter and its configuration can be further altered by pulling or pushing the control element distally or proximally. The radial force of the reconfigurable element can be further controlled via the supportive element and the control element. If the self-expandable compartment is configured to expand more than the diameter of the blood vessel, this self-expansion process may exert too great a force on the wall. This may result in injury to the vessel leading to tearing or perforation. Accordingly, it would be beneficial to be able to control the configuration of the reconfigurable element (including a radial force, size and shape of the reconfigurable element) in a delicate manner to achieve safe and efficient treatment. In addition, the reconfigurable element may need to be further expanded or the radial force of the reconfigurable element increased after achieving its nominal self-expanded diameter. For example, the radial force of the reconfigurable element may need to be enhanced to substantially engage, cutting through, and/or mobilize the occlusion/clot. Therefore, it is expected that the configuration of the reconfigurable element would need to be dynamically changed during the treatment procedure. The device according to the invention is designed to provide such dynamic control of the reconfigurable element.

Alternatively, the reconfigurable element may not be self-expanding and thus the entire opening and closing of the reconfigurable element may need to be controlled. In such a case, when the expandable compartment is unsheathed from the microcatheter to treat the condition in the lumen, the control element may be slightly pulled proximally so that the reconfigurable element may be axially expanded. Similarly to the self-expanding reconfigurable element, further expansion or collapse of this non self-expanding reconfigurable element would be controlled by movement of the control element attached to the supportive element.

In some embodiments, the device comprises at least two mechanisms to control the configuration of the reconfigurable element. First, there is a control provided from the supportive element. The supportive element is generally controlled by the control element. Upon the distal movement of the control element, the supportive element also becomes extended along the axial axis. When the control element retreats proximally, the supportive element would become radially expanded, which would provide further supporting pressure to the reconfigurable element. As the control element moves more proximally, the supportive element would become more expanded and thus the angle between the control element and the supportive element (shown as α in Figure 3 and 4) would be increased. The degree α may range between about 0 to 90 degree. In addition, the configuration of the reconfigurable element (e.g. the overall shape, axial length and outer diameter of the reconfigurable element would be controlled by movement of the control element. Distal movement of the control element would cause the reconfigurable element to shift to its collapsed status, i.e. the reconfigurable element becomes axially extended and its outer radius is reduced while the axial length is increased. Proximal movement of the control element would shift the configuration of the reconfigurable element toward its expanded status, i.e. the reconfigurable element becomes radially expanded. As a result, the axial length of the reconfigurable element may be reduced while the outer diameter is increased. This shift of the reconfigurable element toward its expanded status would enhance the radial force of the reconfigurable element.

Connectors can be metal hypo-tubing, such as stainless steel (SS), Platinum, Gold, Nitinol tubing, plastic tubing such as Polyimide tubing, or can be a segment of coil made from SS, Platinum alloy, Gold, or CoCr alloy wires etc. When using radio opaque material such as Gold, Platinum etc, the connector can also serve as marker.

The configuration of the reconfigurable element would be controlled in the blood vessel in order to remove an occlusion/clot from the lumen and/or expand the lumen in some embodiments. Once the occlusion/clot is engaged by the reconfigurable element, the device would be withdrawn from the lumen and eventually from the body. When the device is withdrawn from the lumen, the expandable compartment that is engaged with the occlusion/clot may be partially withdrawn back into the microcatheter or left distal to the microcatheter. The expandable compartment with occlusion/clot and microcatheter can be simultaneously pulled back into a guiding catheter that has larger inner diameter. The relative position among the micro-catheter, guiding catheter, and the expandable compartment can be determined using fluoroscopy.

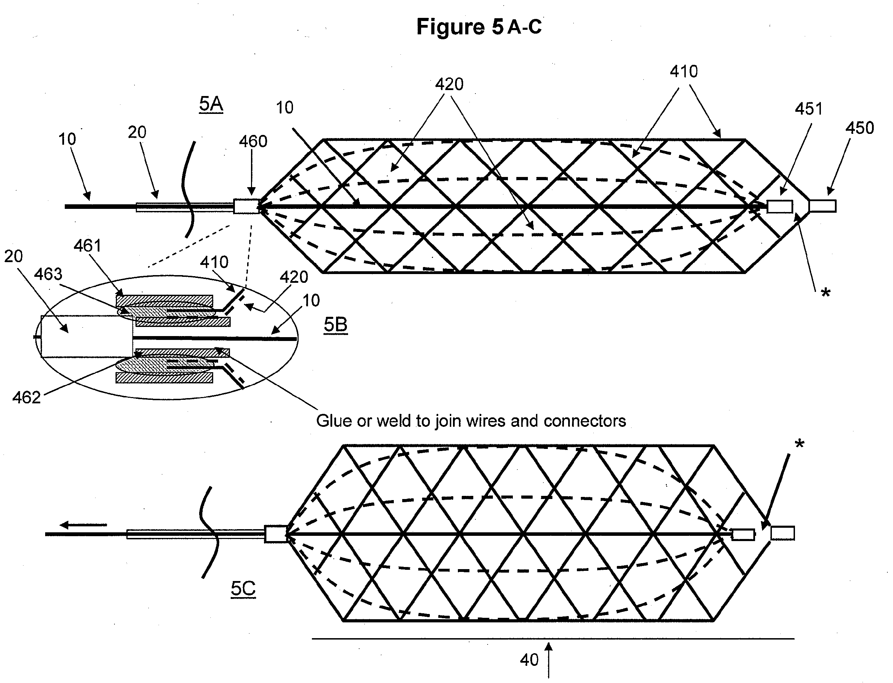

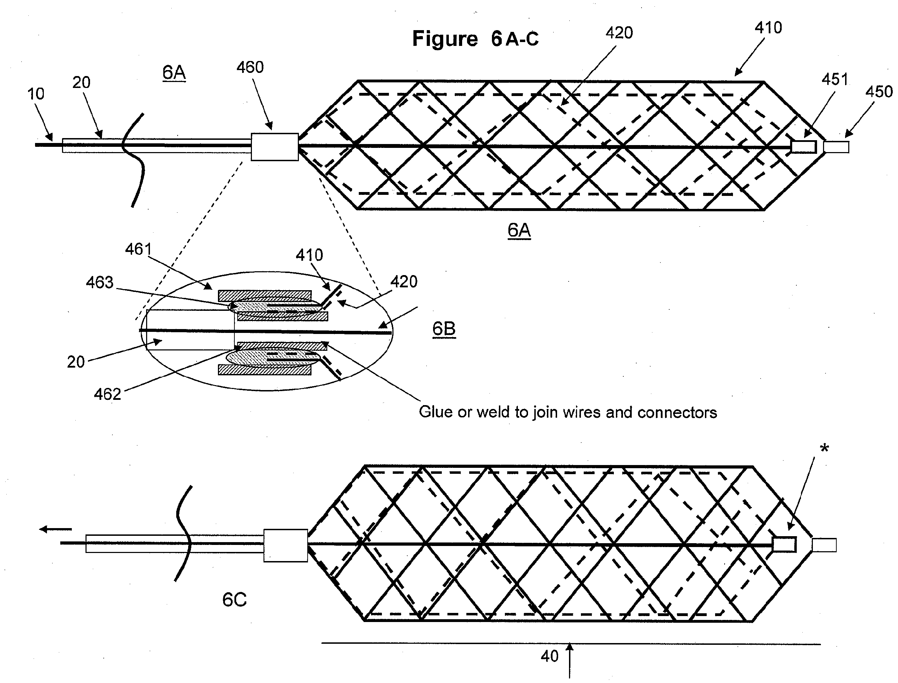

An alternative embodiment of the device is provided in Figure 5 and 6. In this particular embodiment, the supportive element (420) comprises a plurality of wires as shown in Figure 5, or braid structure as shown in Fig 6. The supportive element (420) extends from the proximal end of the reconfigurable element or wire/strut (410) and ends before the distal end of the reconfigurable element (410) (as marked with "*" in Figures 5 -7), thereby forming a double-layered expandable compartment. The distal end of the supportive element (420) may be fixed with the distal tip of control element (10).

As seen in Figure 5B and 6B, association of the reconfigurable element (410) with the supportive element (420) may be done via a proximal connector (460). The proximal connector may comprise at least two compartments, an outer proximal connector (461) and an inner proximal connector (462). The proximal ends of the reconfigurable element wire and the proximal ends of the supporting element are fixed with adhesive, welding, soldering (463), or through mechanical joining in between proximal outer and inner connectors. In some other embodiments, these inner and outer proximal connectors may be a tubular or coil structure and the control element may freely slide through the inner connector.

In the above device, when the control element (10) retreats proximally, it may cause expansion of the supportive element (420), providing support to the reconfigurable element wire/strut, which leads to enlargement of the diameter and enhancement of the radial force of the reconfigurable element (410).

A further alternative embodiment of the device is provided in Figure 7. In this particular example, the supportive element (420) is configured to comprise two plateau positions (480) in which the supportive element (420) may provide the maximal strength of support to the reconfigurable element wire/strut (410). The supportive element (420) may be formed into a sinusoidal shape as seen in Figure 7. The distal end of the supporting element is fixed to the control element (10). Both the proximal ends of the reconfigurable element and supporting element are fixed to the proximal connectors as shown in Fig. 7B. As shown in Figure 7C, the middle (thin) section of the supporting element is connected via middle outer and inner tubing connecters, with control element moving freely inside the proximal and middle connectors (430).

One advantage of having two or more plateaus in the supportive element is that the radial force can be selectively enhanced at preferred positions. As readily seen in Figure 7, the reconfigurable element (410) would receive the maximum strength of support from the supportive element (420) at two plateau positions (480) and the strength of the support would reduce as it is distant from the plateaus. Accordingly, the device can provide a wider range of radial force to the luminal area if desired. Moreover, the number of supportive element can also vary from two to more which may be circumferentially distributed around the control element (10) to vary the radial force and/or the outer shape and density of the device.

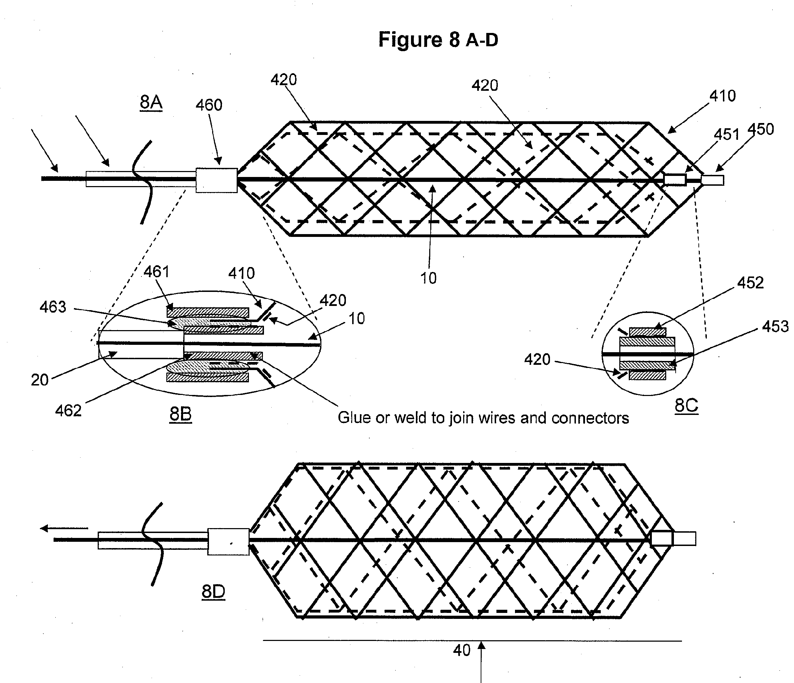

A still further alternative embodiment of the device is provided in Figure 8. In this particular example, the control element (10) runs through the distal end of the supportive element (420) and reaches to the distal end of the reconfigurable element (410). The distal end of the supportive element may freely slide along the control element as seen in Figure 8C. The supportive element (420) may be associated with the control element (10), for example, via a connector (451) at the distal end of the supportive element (420). The connector 450 comprises of a supportive distal inner connector (453) and supportive distal outer connecter (452), joining the distal end of the supportive elements in between them. The control element (10) can slide in the lumen of the inner connector. When the control element (10) is pulled proximally, the distance between the connector (450) of the reconfigurable element and the distal end of the supportive element (451) become shorter. When the two connecters are in contact to each other, the supportive element will be expanded, pushing against the reconfigurable element, and generating additional radial force as seen in Figure 8D. One benefit of this particular embodiment of Figure 8 would be that the distal end (451) of the supportive element and the distal end (450) of the reconfigurable element are aligned to the axial direction, avoiding tilting of the supportive element tip while the control element is pulled proximally. It may also avoid constraint in the wire axial length between the reconfigurable element (410) and the supportive element (420) when the device is retracted into the microcatheter.

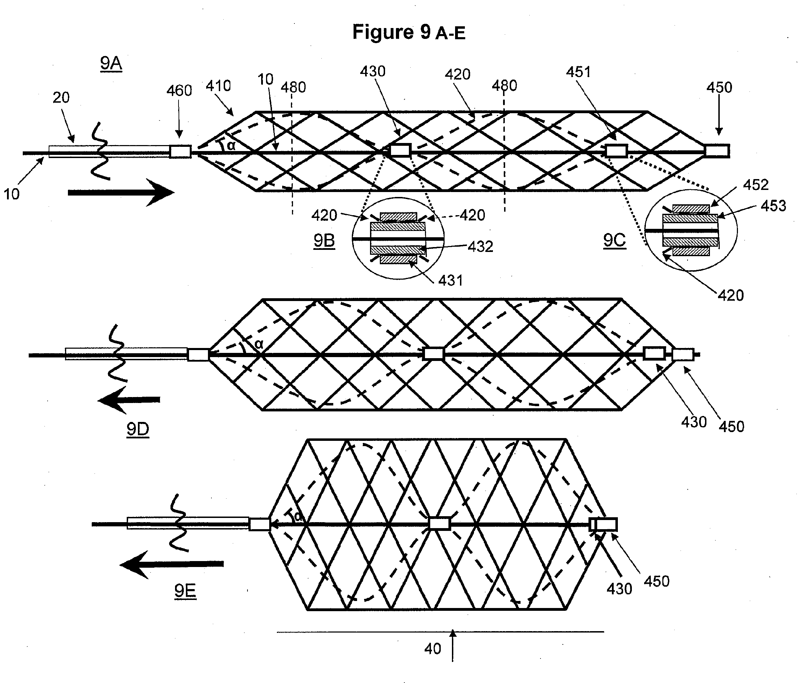

A still further alternative embodiment of the device is provided in Figure 9. In this particular example, the supportive element (420) comprises at least two plateau positions (480). Moreover, the distal end of the supportive element (420) may not be substantially immobilized at about the distal end of the device. Therefore, the supportive element (420) may be associated with the control element (10), for example, via connectors (430) at the distal tip of the supportive element. These connectors (430) may be configured to freely slide along the control element. The structure of the connector (430) also comprise of inner and outer connectors to ensure control element can move freely through the connectors. Accordingly, when the control element is pulled proximally, the reconfigurable element distal connector (450) may move closer to the distal end of the supporting element. When the two connecters are contacted to each other, the supporting element will be expanded, and will push against the reconfigurable element (410), generating additional radial force as seen in Figure 9E,

This particular device of Figure 9 may provide at least three benefits. The supportive element comprising more than one plateau position which may allow the radial force to be selectively enhanced at preferred positions. Accordingly, the device can provide a wider range of radial force to the luminal area if desired. In addition, similar to the device of Figure 8, the distal end of the supporting element and the distal end of the supporting element is aligned to the axial direction by the control element, avoiding tilting of the tips while the control element is pulled proximally. It can also avoid constraint in the wire axial length between the reconfigurable element (410) and the supportive element (420) when the device is retracted into the microcatheter.

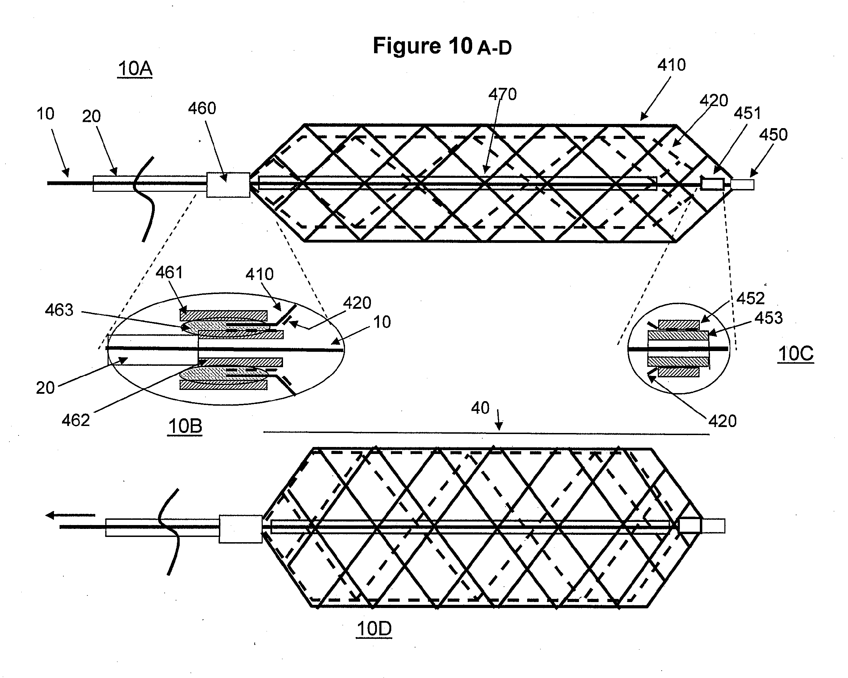

An adjustment tube may be utilized in the device in all the described designs. As seen in Figure 10, the adjustment tube (470) may be placed over the control element between the proximal and distal ends of the supportive element. The adjustment tube may optionally be freely slide along the control element. While the adjustment tube is present in the device, it may prevent the connector (451 or 430) to be too close to the proximal ends of the expandable compartment. Accordingly, the device with the adjustment tube (470) may prevent excess axial expansion of the reconfigurable element. The tubing can also prevent or reduce friction between the control element and the supportive element struts/wires when pulling the device back into an introducer sheath or a micro-catheter.

A still further alternative embodiment of the device is provided in Figure 11. In this particular umbrella-shaped device, the reconfigurable element (410) may comprise a plurality of wires and form a tubular structure as seen in the figure. The supportive element, which may also comprise a plurality of wires, can be used to alter the configuration of the reconfigurable element. The supportive element (420) may be associated with the control element (10) as well as the reconfigurable elements (410) and at least some of the association positions may be coupled with markers (440). The supportive element (420) as well as the reconfigurable element (410) may be associated with the control element (10) in a substantially immobilized manner. All wires of the supportive element (420) may be associated with the control element (10) via a connector (430). The connectors (430) are fixed to the control element. Therefore, pulling the control element (10) proximally or pushing it distally may also act on the other wires (i.e. the reconfigurable element and the supportive element) accordingly.

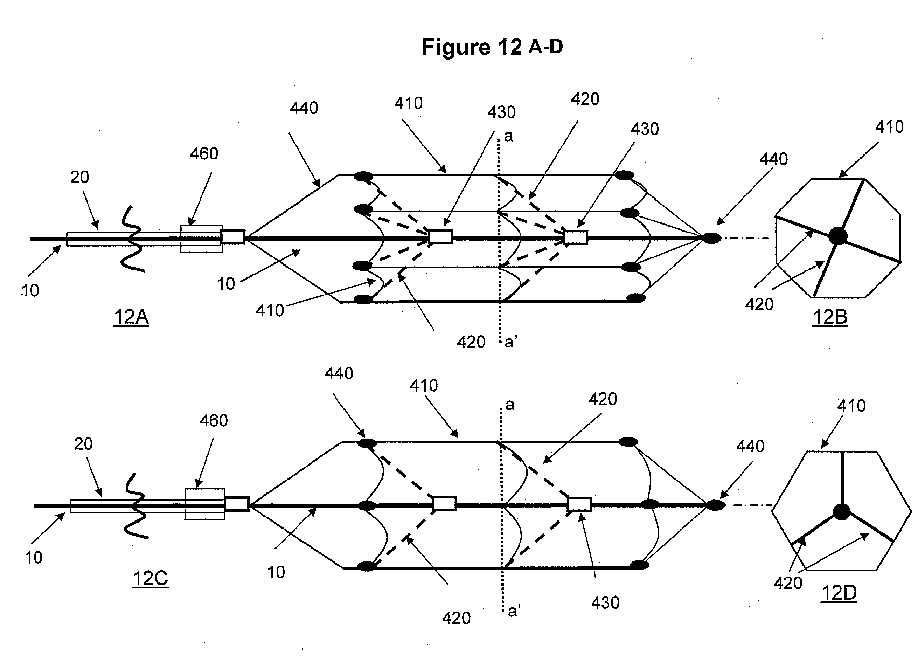

Some non-limiting and illustrative alterations of the foregoing device are shown in Figure 12. In the device depicted in Figure 12A and B, the reconfigurable element (410) comprises 8 generally linear wires aligned along the axial axis of the device. The device further comprises 2 sets of the supportive element (420) distributed between the proximal and distal ends of the device. Each of this set of the supportive element (42) may comprise 4 wires to manipulate the radial force of the device. Alternatively, the device of Figure 12C comprises the reconfigurable element (410) comprising 6 generally linear wires aligned axially and the supportive element (420) comprising 3 wires in each set. In addition, any further and other alterations such as using 1 or 2 set of the supportive element as well as using more than 3 sets of the supportive element can be applied to the device. Moreover, the reconfigurable element and the supportive element may be in a form of wire mesh (braid) similar to those seen in Figure 7, if desired.

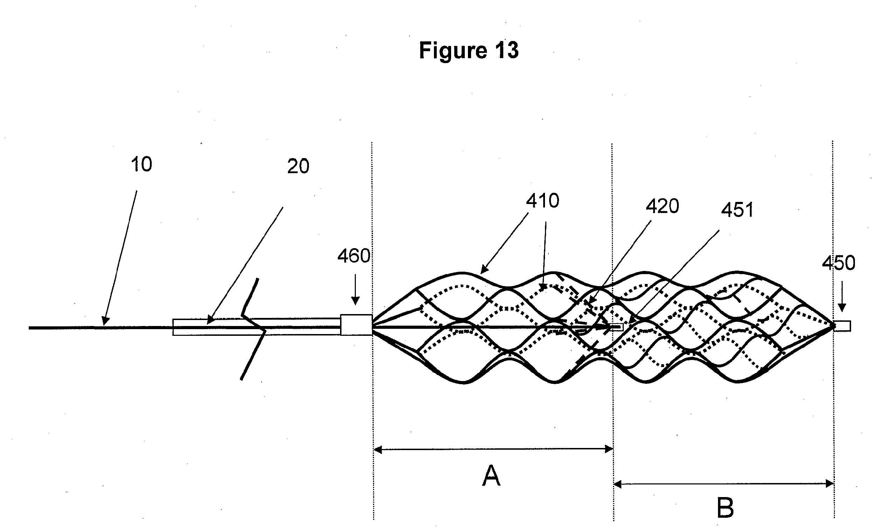

Another embodiment is illustrated in Figure 13. In this particular example, the size of reconfigurable element cell and strut size or diameter of wire can be varied within a single device. From the proximal to distal end of the reconfigurable element, the cell may change from large size to small size, or vice versa, and the strut size may change from thick to thin strut, or vice versa. The device illustrated in this figure, the zone A has generally larger cells in the reconfigurable element (410) than those in the zone B, or vice versa. Further, the strut size or diameter of wire can be thicker in the zone A as compared to that of the zone B, or vice versa. Advantages of these embodiments may include at least one or more of the following:
1) The large proximal cell size may have less wire density which can increase the force and pressure each strut exerts when the radial force is increased. This may enable the reconfigurable element (wire or strut) to cut through or break occlusion/clot more easily. An occlusion/clot may also fall into the expandable compartment more easily because of wider opening. The small sized distal cell is to catch and hold the occlusion/clot debris that are broken from the proximal end of the expandable compartment, so debris would not escape from the device and go to down stream.
2) The strut size of the reconfigurable element can also change from the proximal end to distal end, with wide/thick strut at the proximal end and thin struts at the distal end. The large and strong proximal end strut would have grate stiffness and can cut the occlusion/clot more easily. Due to the increased number of cells at the distal end the strut size at the distal end may need to be thin to enable the device to keep a small profile in its compressed state to be able to fit in a microcatheter.

Accordingly, the examples shown in the application should not be considered to limit the scope of the invention and many different modifications and alternations, which should be obvious to a person with ordinary skill in the art, can also be done without affecting the scope of the invention.

The device can be manufactured by a variety of techniques that are known in the art. For example, the reconfigurable element and the supportive element can be fabricated from the some piece of material by laser-cutting a hypo-tube. The hypo-tube after being cut by a laser may be heat set to a desired shape and size of the reconfigurable and the supportive components, which can be further assemble into a device as illustrated in Figure 13.

Alternatively, reconfigurable element/struts and supportive elements/struts can be made form the same thin sheet by laser cutting or photo etching as seen in Figure 14A. The component may be heat set to a desired shape and size of the reconfigurable and supportive components. These components may be further assembled into a device. The sides of the reconfigurable element can either be joined using adhesive, welding, soldering, and mechanical joining etc. to form a close-sided-expandable compartment (see Fig. 14B), or simply left open as open-sided-expendable compartment (Fig. 14C).

The distal end of reconfigurable element can either be close-ended (wires or struts ends are joined, shown in Figure 14B and 14C), or can be open-ended (i.e. wires or struts ends are not joined, as shown in Fig. 14D).

Both the hypo-tube and metal sheet may be made of one or more selected from the group consisting of nickel-titanium (NiTi) alloy, stainless steel, titanium (and its alloys), and cobalt chrome (CoCr) alloys etc.

One advantage of many embodiments of the above-listed techniques of processing the hypo-tube or the thin sheet is that association (or joining) of multiple wires (e.g. between the reconfigurable element and the supportive element) may be avoid, so the device profile (size) may be small. These embodiments compare favorably to braid wire structures, since the struts of the reconfigurable element are all connected at the corners of each cell unit or window. The radial force can be controlled through cell shape and structure design without increasing the profile of the device.

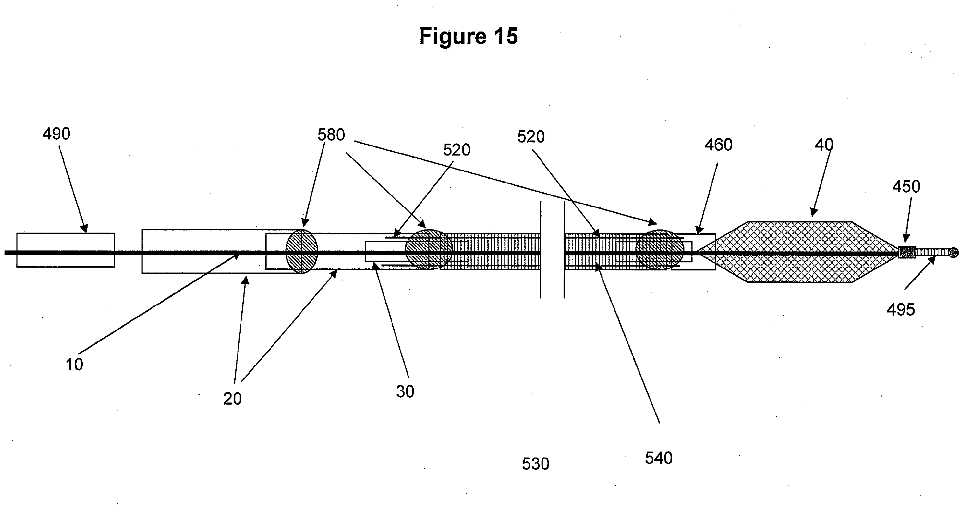

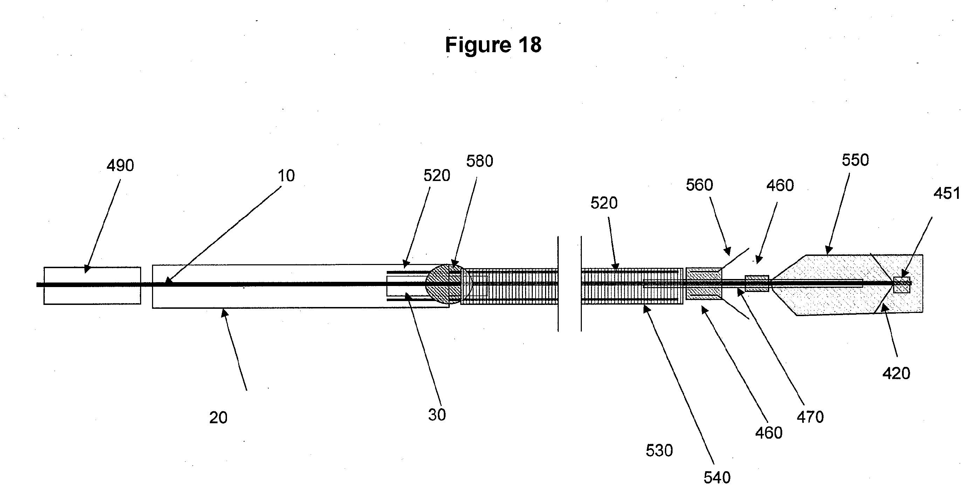

The device is designed to place the expandable compartment in the vasculature of a subject. The subject can be a patient who is in need to treatment such as removing blood occlusion/clot and/or recovering blood flow in body. An exemplary, non-limiting embodiment of the device is illustrated in Figure 15 and 18. According to some aspects, there is a coil section (540) in between the pusher tubing (20) and the expandable compartment (40). This coil section can be considered as a part of the pusher tubing. The function of the coil (540) can make the distal section of the device flexible, so the device can pass through tortuous vessels. To further improve the device pushability, plastic tubing, such as PTFE, PET etc. may be added around the coil, or simply replace the coil as a flexible pushing component at the distal end of the pusher tubing. If the coil is used, one or more than two thin wire (520) may be used to link the connector of the expandable compartment (40) and the pusher tubing (20) to prevent the coil (540) from stretching. According to further some aspects, a pusher tubing (20) may be connected to the coil (540). Further, the control element (10) can slide freely, in the lumen of the pusher tubing (20), as well as in the coil or flexible tube section and the proximal connecter (460) of the reconfigurable element. According to still some other aspects, at the proximal end of the device, a control element handle tubing (490) may be added and fixed to the proximal end of control element (10). With this feature, an operator can easily grab the control element handle tubing (490) to control the opening or closing of the reconfigurable element. All the connections between parts can be joined through gluing (adhesive), welding, soldering, or etc. Referring to Figure 15, a flexible coil (495) may be added to the distal end of the expandable compartment to make the device tip atraumatic, avoiding poking the vessel lumen.

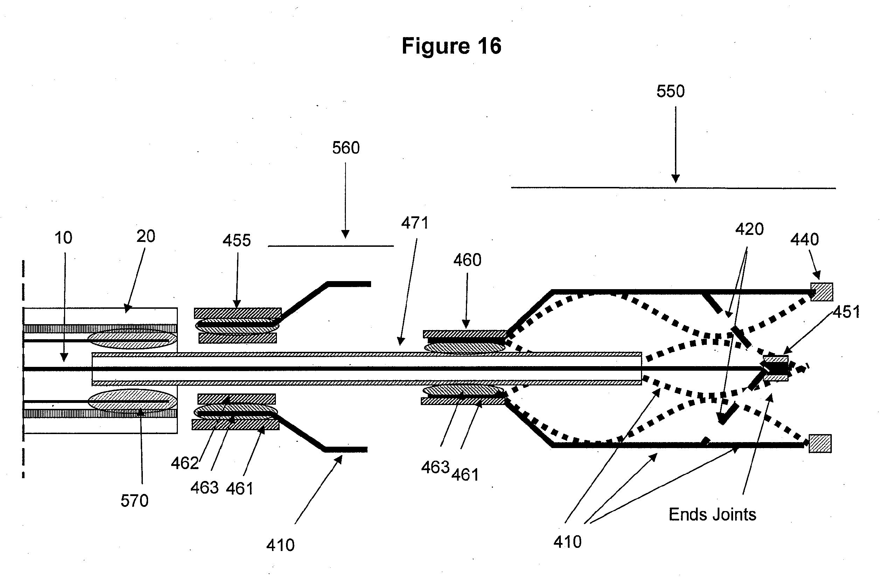

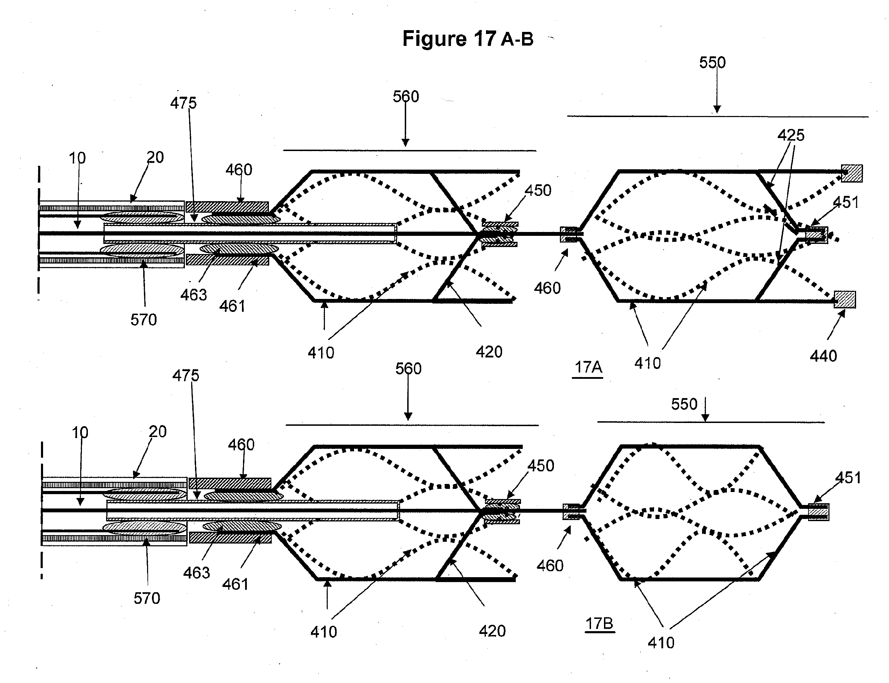

In Figure 16 and Figure 17, further alternative embodiments are illustrated. In these embodiments, the device may comprise two structures, a distal expandable structure (550) and a proximal expandable structure (560). For the device shown in Figure 16, The distance between the two expandable structures can be changed/adjusted, i.e. the distal structure may be pulled/slide toward the proximal structure or pushed/slide away from the proximal structure, for example by pushing and pulling the control element (10).

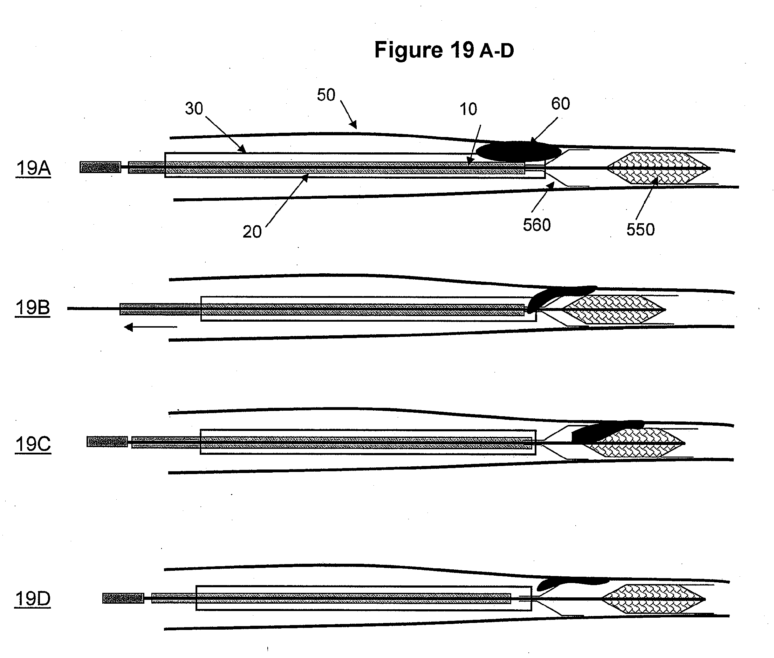

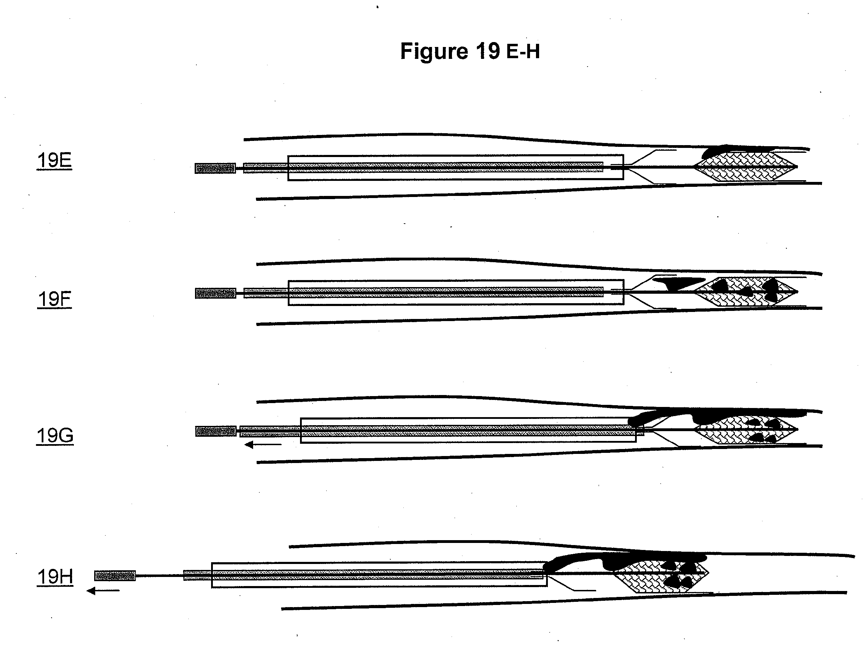

Referring to Figure 16, the distal structure (550) is in a form of basket or expandable compartment comprising a reconfigurable element (410), a supportive element (420), and a control element. The distal tip of the control element (10) can be connected to the supportive element (420), and move freely inside a sliding tube (471) and pusher tubing (20). By adjusting the supportive element through the control element, the radius and radial force of the distal structure can be adjusted. As illustrated elsewhere in the application, adjustment of the supportive element can be achieved by pushing or pulling the central element. In some other embodiments, the proximal end of the sliding tube (471) can be fixed to the distal end of the pusher tubing. The distal end of the distal structure (550) can be fixed to a middle point of the sliding tube via a connector (460). The proximal structure may be an umbrella-shaped component (560). Its proximal end may be associated with a connector (455) which may comprise outer and inner connectors. The inner ID of the connector is generally larger than the outer diameter of the sliding tube, so that the sliding tube can slide freely in the connector. During occlusion/clot retrieving process, while unsheathing the microcatheter, the proximal structure is held by the tip of microcatheter due to fiction. It may be separated from the distal structure. When pulling the pusher tubing, the distal structure moves toward the proximal structure. An occlusion/clot in between the two structure can be engaged/grabbed (the detailed mechanism of how the device catches occlusion/clot will be further illustrated in Figure 19). If pulling the control element proximally, the distal structure may be further expanded. The segment of the sliding tube (between connector 460 and tip of the sliding tube) has the same function as adjustment tube (470) in Figure 10, i.e. to prevent over expansion of distal structure as illustrated in previous section. Markers (440) can also be added to the distal tip of the distal and proximal structures as needed.

Referring to Figure 17A, the proximal structure (560) is an expandable compartment, comprising of reconfigurable element (410), supporting element (420) and control element (10). The proximal end of this structure can be joined in between the outer tube (460) and an inner tube (475). The distal tip of the control element (10) can be connected to the supportive element (450), and move freely in the inner tube (475) at the distal end of the proximal structure, as well as in pusher tubing (20). By adjusting the supportive element through the control element, the configuration and radial force of the proximal structure can be adjusted. The distal structure (550) is in a form of basket or self-expandable compartment comprising a reconfigurable element (410) and enclosing element (425). In the middle of the distal structure, the tip of the enclosing element can be joined by connecter (451) to form a closed compartment/structure. The structure of the enclosing element can be the same as that of the supporting element described previously, but its function is just to close the compartment. Since the tip of the enclosing element is not connected to the control element, configuration and radial of the structure (550) cannot be adjusted. Alternatively, the distal structure can also be built without the enclosing element. The distal end can be closed by joining the distal wires/struts of the reconfigurable element (410) as illustrated in Figure 17B (451). The proximal end of the distal structure (550) may be fixed to the distal tip of the control element. During retrieval process, the distal structure may catch occlusion/clot debris that are unable to be held/contained by the proximal structure. The space between the two structures can also serve as a room to contain occlusion/clot or occlusion/clot debris during retrieval process.

The inner tubing (475) extending from the pusher tubing tip/proximal connector into the middle of the proximal structure (560) has the same function as adjustment tube 470 in Figure 10, i.e. to prevent over expansion of distal expandable structure as illustrated in previous section.

In the embodiments which comprise two compartments, both the distal and proximal components can be made through laser cutting, photo etching, or wire braiding as disclosed in elsewhere of the application. Various materials described elsewhere can be used to make the reconfigurable element. In such embodiments, the strength/stiffness or wire/strut size of the proximal structure can be different from the distal structure. Further, the size of the distal structure (when it is fully expanded) may be different from that of the proximal structure.

A device which is configured to apply the embodiment comprising two expandable structures using design shown Figure 16 as an example is illustrated in Figure 18. In this particular embodiment, the distal structure (550) and the proximal structure (560) are employed in the device. Retrieval of an occlusion/clot using the device according to Figure 18 is illustrated in Figure 19. The mechanism shown in Figure 19 is merely an illustration of various applications, and presented as an illustration of certain embodiments. As discussed elsewhere in the application, the device according to the present application can be used to retrieve or remove occlusion/clot in, for example, a blood vessel. Further, the device can be used to expand the luminal area and/or restore blood flow, which may or may not require retrieval of an occlusion/clot.

In this hypothetical condition where an occlusion/clot (60) is located in a blood vessel (50) as seen in Figure 19A, the microcatheter (30) comprising the retrieval device can be navigated and located in close proximity to the occlusion/clot or distal to the occlusion/clot. The microcatheter (30) then is unsheathed to expose the retrieval device to the occlusion/clot. In some embodiments shown in Figure 19, an occlusion/clot can be engaged and retrieved through the following mechanisms:
an occlusion/clot (60) can be held between the proximal structure (560) and the tip of micro-catheter (30) and removed from the original position (Figure 19B).
an occlusion/clot (60) can be held in between the proximal structure (560) and the distal structure (550) and removed from the original position (Figure 19C).
an occlusion/clot (60) can be held/engaged by the proximal structure (560) and removed from the original position (Figure 19D). In some cases, the occlusion/clot can be engaged between the proximal structure and the artery wall and removed with friction between the occlusion/clot and the device.
an occlusion/clot (60) can be held/engaged by the distal structure (550) and removed from the original position (Figure 19E). In some cases, the occlusion/clot can be engaged between the distal structure and the artery wall and removed with friction.
an occlusion/clot (60) can be broken-up into debris from the proximal structure. They may fall into, or be caught by the distal structure (550) and/or stay in between proximal structure (560) and distal structure (550) (Figure 19F) and removed from the original position.
an occlusion/clot (60) can be engaged at various location/points and removed through combinations of any above mechanisms (Figure 19G) and removed from the original position.

According to some embodiments, during the occlusion/clot engagement and, or retrieval process, the control element (10) can be pulled back at any time to maneuver the radial force and radius (i.e. size) of the retrieval device to ensure that the occlusion/clot is engaged with the device and not slide away from the device (Figure 19H).

Figure 20 illustrates a device according. The device may comprise a tubing component and an engaging compartment. The tubing component may comprise a plurality of tubing elements such as a pusher tubing (20) and connecting tubing (31). The engaging compartment may comprise a distal engaging element (90) and a proximal engaging element (65). The device may further comprise a central wire (10).

As seen in Figure 20, in certain embodiments, the distal engaging element (90) and the proximal engaging element 65 are associated with the central wire (10). In certain some embodiments, the distal engaging element 90 may be connected (or fixed) to the central wire (10), and the proximal engaging element (65) may be connected to the connecting tubing (31), which is also connected to the pusher tubing (20). The central wire (10) may be placed inside the connecting tubing 31 and the pusher tubing (20), and move freely through the connecting tubing as well as the pusher tubing. In some embodiments, the central wire (10) and the pusher tubing (20) may extend to the proximal end of the device.

In some embodiments, the central wire (10) and the connecting tubing (31) may be maneuvered independently, thereby allowing separate control of the distal and the proximal engaging elements. More particularly, the distal engaging element would be controlled by movement of the central wire which is connected to the distal engaging element; and the proximal engaging element would be controlled by movement of the connecting tubing, or pusher tubing, which is connected to the proximal engaging element. In certain embodiments such as those seen in Figure 20, the pusher tubing and the connecting tubing are connected to each other, and thus movement of the pusher tubing may ultimately control the proximal engaging element.

In certain embodiments, the distal engaging element may have an open-end at its distal end (e.g. see Fig. 20A). Alternatively, in certain other embodiments, the distal element may have a closed-end at its distal end (e.g. see Fig. 20C). The distal engaging element (90) may be connected to the central wire (10). The distal engaging element may be connected at about its proximal end to the central wire by various means, e.g. welding, gluing or clipping.

In certain embodiments, the connection between the central wire (10) and the distal engaging element (90) is placed in the distal element connector (80). The distal element connector may be in form of a short tube or coil and placed over the central wire (10). In some embodiments, the distal element connector may comprise an outer distal element connector (82) and/or a distal element joining media (81). In such embodiments, the proximal end of the distal engaging element may be placed in between the outer connect and central wire. On some occasions, the distal engaging element, connector and the central wire may be connected by joining media such as a clip, clasp or fastener which fastens or holds the distal engaging element and the central wire together.

According to some embodiments, the proximal engaging element may comprise a plurality of wires. Alternatively, the proximal engaging element may be made from a tubing, e.g. by a laser cutting technique. Therefore, in certain embodiments, a small segment of the tubing is saved at the proximal end and the struts are cut or formed at the distal end as seen in Figure 20A'. In such embodiments, the tubing segment at the proximal end that is an integral part of the proximal engaging element may serve as a proximal element connector. In alternative embodiments, a separate proximal element connector may be added to the device as seen in Figure 20A. In some embodiments, the proximal element connector (41) may comprise a plurality of elements, such as joining media (42) and an outer proximal element connector (43). In such embodiments, the proximal end of the proximal engaging element may be placed at the inner proximal element connector, at the outer proximal element connect or between the outer proximal element connectors and the tip of the connecting tubing. In some embodiments, the proximal end of the proximal engaging element is placed in the inner proximal element connector. In some occasions, the proximal end of the proximal engaging element and the connector may be jointed using media such as a clip, clasp or fastener which fastens or holds the proximal engaging element, more particularly the proximal end of the proximal engaging element. Alternatively, the proximal element connector may comprise a single layered tubing in which the proximal engaging compartment is placed.

When a separate proximal element connector is present (e.g. Figure 20), the connection of the proximal engaging element to the proximal connector can be done via various means, such as welding or gluing. Alternatively, as discussed above, the proximal connector may comprise a joining media which can fasten or hold the proximal engaging element. In any event the proximal element connector surrounds the central wire and allows the free movement of the central wire inside the proximal element connector.

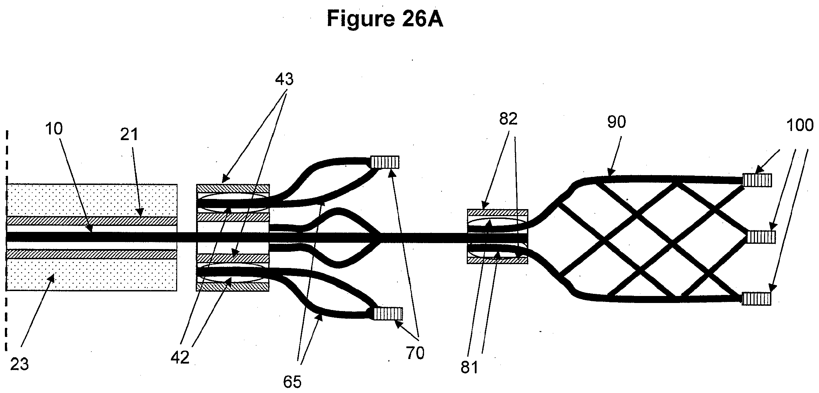

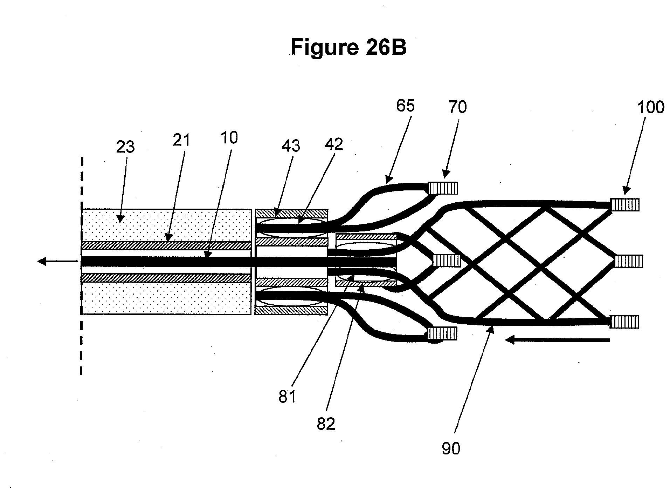

In certain some embodiments, the device contains a connecting tubing which is connected to the proximal engaging element. In some embodiments, the connecting tubing may be further connected to the pusher tubing, e.g. as illustrated in Figure 20. With this configuration, the proximal engaging element is permanently connected to the pusher tubing. However, alternatively, fixation of the proximal engaging element to the pusher tubing is temporal or reversible, and thus the proximal engaging element, optionally along with the proximal connector, can be dissociated from the pusher tubing if desired. As a further alternative, the proximal engaging element and the proximal element connector associated thereto may not be fixed at the tubing compartment (see, e.g. Figure 26). Instead, they can freely slide along the central wire.

According to one aspect of the invention, the position of the distal engaging element can be determined by controlling the central wire. For example, in the embodiment illustrated in Figure 20, the distal engaging element is connected with the central wire. An operator is able to control the movement of the central wire (e.g. pushing in and out) to locate the distal engaging element in a desired position. In certain embodiments, the proximal engaging element may be positioned via movement of the tubing element, e.g. a pusher tubing, a connecting tubing or both. As illustrated in Figure 20, in some embodiments, the proximal engaging element is fixed at about the distal end of the tubing element(s). Accordingly, an operator can locate the proximal engaging element in a desired position by controlling the tubing element, i.e. a pusher tubing and connection tubing. With these configurations, the two engaging elements can be maneuvered independently. Further, it allows that the distance between the two engaging elements can be varied. This aspect of the device where the distance between the two engaging elements is variable is beneficial when treating patients with the device. This design has the ability to engage an occlusion or clot firmly by holding it between the two separate engaging elements. Further, by adjusting the position of each engaging element, and the distance therebetween, it can maximize the efficiency and accuracy of the holding.

In Figure 20B, a different status of the device illustrated in Figure 20A is illustrated. In this closed position, the central wire is pulled proximally and thus the distal engaging element is pulled toward the proximal engaging element, shortening the distance between the two engaging elements.

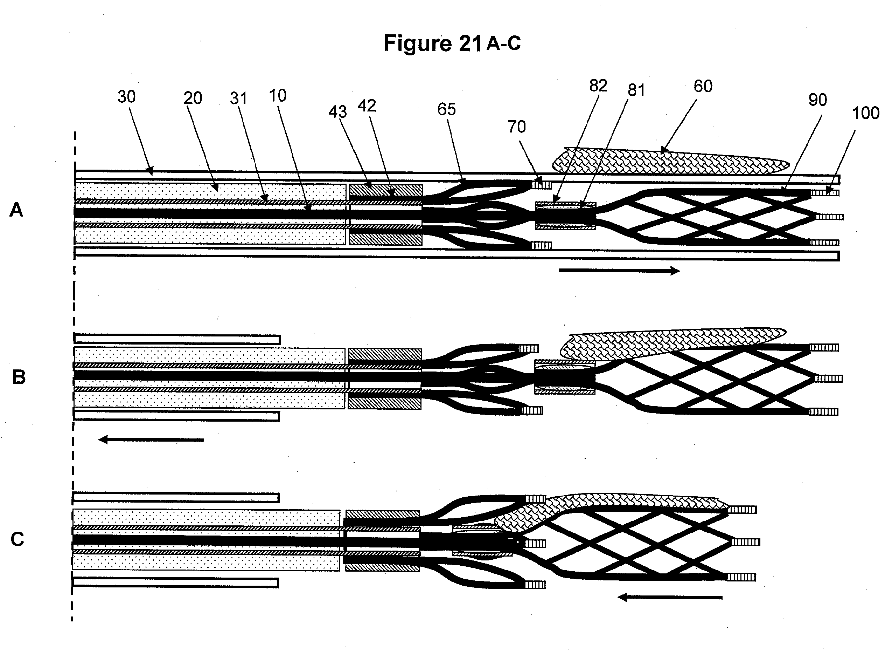

Figure 21 shows an illustrative embodiment of removing a clot or occlusion from a body lumen (e.g. blood vessel). In certain embodiments, the device may be used to engage a clot or occlusion and remove the same from the body lumen as follows:
(A) A microcatheter (30), with guidance of a guide wire is first disposed to an area where an occlusion occurs in a body lumen. Depending on hardness, length, location, and shape of the clot or occlusion, the microcatheter may partially penetrate the clot (or occlusion), or pass distal to the clot. Following this, the tubing compartment and the engaging components may be delivered to the occlusion through the microcatheter. In this technique, the proximal expendable element is separated from the distal expendable element. When placing the device, the tip of the proximal engaging element is placed behind/proximal to the clot or occlusion
(B) The microcatheter pulled back to unsheath the engaging components and part of the tubing compartment. Ideally, the proximal engaging element is proximal to the clot, and the distal engaging element is distal to the clot, or distal to the proximal portion of the clot, if the clot is substantially long. In some embodiments, the clot would be held or engaged at least in part by the distal engaging element at this stage as seen in the Figure 21B.
(C) An operator can further adjust the position(s) of one or both of the engaging elements if desired. For example, while holding the pusher tubing to fix the proximal engaging element, the operator can proximally pull the central wire that is connected to the distal engaging element. Then the clot would be moved proximally due to the friction between the distal engaging element and the clot. As this occurs, the distance between the two engaging elements is shortened, and the clot is grabbed or engaged in and/or between the two engaging elements. If the operator feels the resistance while pulling the central wire and the distal engaging element, this may indicate that the clot is engaged in and/or between the two engaging elements. He or she can then lock the positions of the distal and proximal engaging elements and pull the device and the engaged clot, together with the microcatheter, from the body lumen (e.g. an artery). In some embodiments, the location of the device can be identified by markers (70) and/or (100) placed on the device.

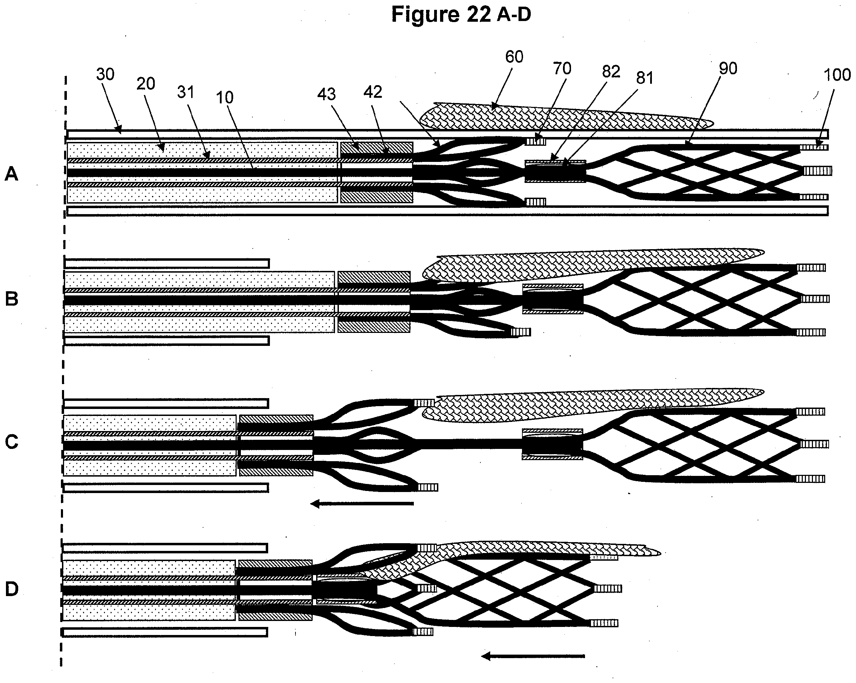

In Figure 22, an alternative embodiment or technique of removing a clot or occlusion from a body lumen is illustrated. The device may be used to engage a clot or occlusion and remove the same from the body lumen as follows:
(A) A microcatheter (30) is navigated to an area where an occlusion occurs in a body lumen. The microcatheter may be advanced over the distal end of the clot or occlusion. Depending on hardness, size, location and shape of the clot or occlusion, the microcatheter may penetrate the clot (or occlusion), or pass by the clot without substantially disturbing the clot. In some embodiments, the tubing compartment and the engaging compartment are introduced into the microcatheter once the microcatheter is in position near the occlusion area. In certain occasions, an operator may dispose the microcatheter and the expendable compartment in a position such that once unsheathed the proximal engaging element is distal to the proximal end of the clot. As explained elsewhere in the application, the location of the device can be monitored, for example, by using the markers present in the device.
(B) The microcatheter is unsheathed, and the engaging compartment and part of the tubing compartment are exposed.
(C) The operator can further adjust the position(s) of one or both of the engaging elements. For example, while holding the central wire and consequently the distal engaging element stable, pulling the proximal engaging element back until its distal end just passes the proximal end of the clot. This can be indicated by viewing the markers (70) in the distal end of the proximal engaging element. The distal end of the proximal engaging element is open at this position. Alternatively, if desired, the distal engaging element can be adjusted while the proximal engaging element is on hold. Further, if desired, the proximal and distal engaging elements may be adjusted together to ensure the engagement of the clot.
(D) While keeping the proximal engaging element stable by fixing the pusher tubing, an operator can pull the central wire that is connected to the distal engaging element. Then the clot may be moved proximally due to the friction between the distal engaging element and the clot. The distance between the two engaging elements is shortened, and the clot is grabbed or engaged in and/or between the two engaging elements. If the operator feels the resistance while pulling the central wire and the distal engaging element, it may indicate that the clot is engaged in and/or between the two components. He or she can then lock the positions of the distal and proximal engaging elements and pull the device and the engaged clot, together with the microcatheter, from the body lumen (e.g. an artery). In some embodiments, the location of the device and microcatheter can all be identified by markers (70) and/or (100) placed on the device.

Some advantages of the embodiment illustrated in Figure 22 include that the proximal engaging element can be positioned proximal to the clot after unsheathing, to ensure relatively high precision in the placement of the engaging component with respect of the clot or occlusion. Because the proximal engaging element can be put immediately behind the clot or occlusion, when pulling the distal engaging element to move the clot proximally, the clot only needs to travel for a very short distance before it is engaged between the two engaging elements. Thus the chance of the clot being lost is reduced as compared to the technique shown in Figure 21.

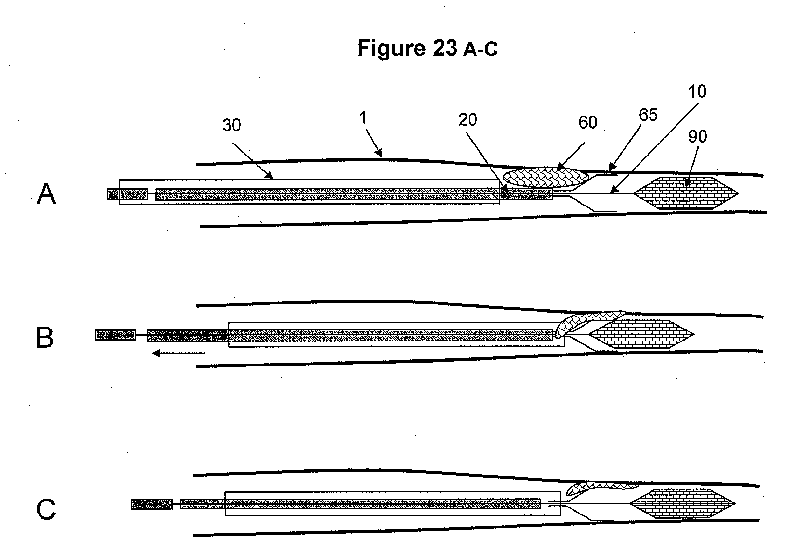

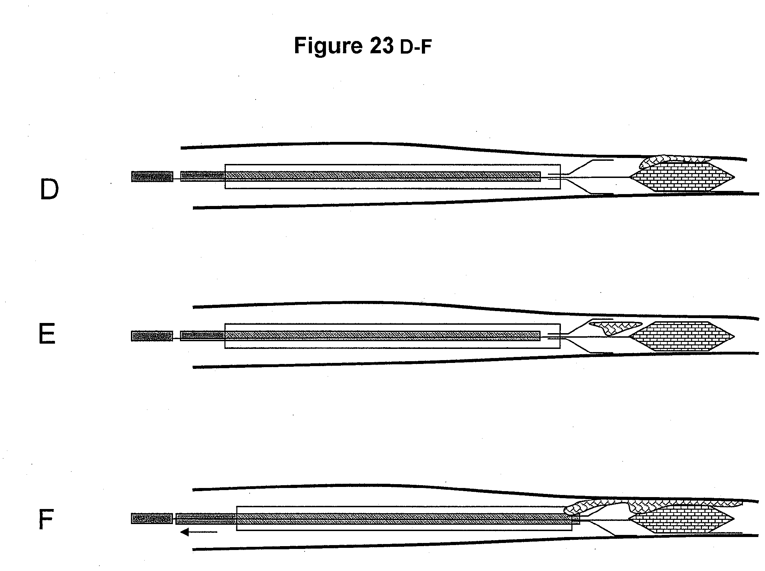

In Figure 23, some additional mechanisms of engaging the clot using the device are presented. As depicted in Figure 23, the clot may engaged between the tubing element and the body lumen surface (e.g. an artery wall)(23A), between the microcatheter and the proximal engaging element (Figure 23B), between the proximal engaging element and the artery wall (23C), between distal engaging element and artery wall (23D), between the proximal and distal engaging elements (23E), and between proximal engaging element and the microcatheter tip and at the same time between the two engaging elements (23F).

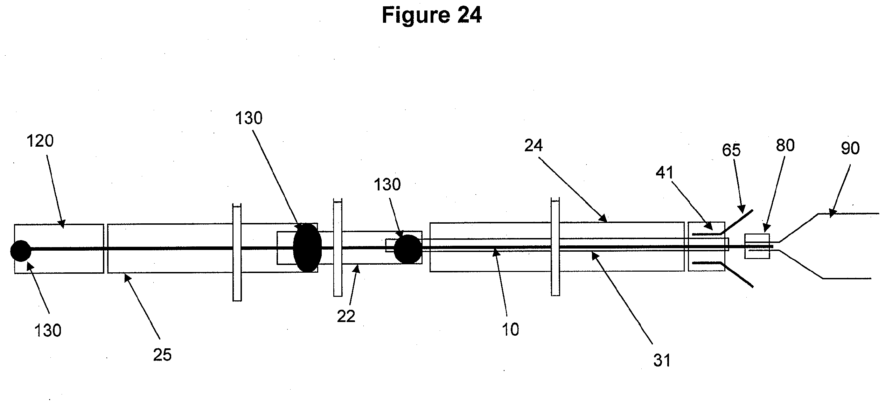

Figure 24 illustrates an illustrative embodiment of a system comprising the foregoing device. In some embodiments, the central wire (10) can extend to the proximal end of the device, and be jointed to a distal component handle (120). The length of the system from its distal end to proximal end may be approximately 100 to 200 cm. In some embodiments, the length of the system from its distal end to proximal end may be approximately 100 cm, 110 cm, 120 cm, 130 cm, 140 cm, 150 cm, 160 cm, 170 cm, 180 cm, 190 cm, and 200 cm. In some other embodiments, the length of the system from its distal end to proximal end may be shorter than 100 cm or longer than 200 cm. In some embodiments, the system may comprise a plurality of pusher tubing such as a proximal pusher tubing (25), a middle pusher tubing (22), and a distal pusher tubing (24). In some other embodiments, the connecting tubing may be connected to the middle and proximal pusher tubings. Both the distal and proximal engaging elements can be operated at the distal end of the system, i.e. with the distal component control handle and the proximal pusher tubing as illustrated in Figure 24, by an operator.

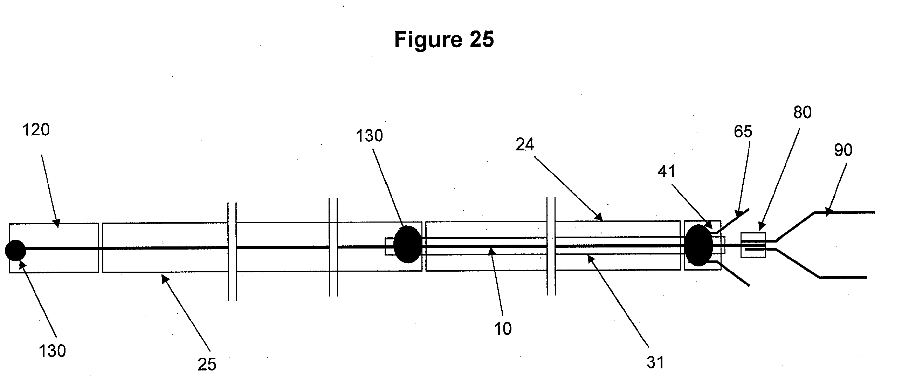

Figure 25 illustrates an alternative embodiment of a system in which a distal pusher tubing and a proximal pusher tubing are present. On top of the connection tubing, there is a distal pusher tubing. In some embodiments, at least some part of the distal pusher tubing is flexible to ensure the device being able to navigate through tortuous path. In other embodiments, at least some part of the distal pusher tubing may be lubricious and flexible, and thus it also enhances pushability of the device. In certain embodiments, the proximal end of the pusher tubing can be made from a stiff/firm tubing to enhance the device pushability.

In case the device needs to be pulled back into the microcatheter, each of the engaging elements can be operated separately. For example, the proximal engaging element can be first pulled back into the microcatheter, and then the distal component can be pulled into the microcatheter by pulling the central wire avoiding the two elements (and their struts) being overlapped. This will avoid the two expendable elements overlapping each other and not being able to be pulled into the microcatheter. In certain embodiments, the positions of the two engaging elements may be locked once the clot is engaged. In such embodiments, movement of the two engaging elements would be synchronized and their introduction into the microcatheter would be in succession.

Figure 26 illustrates an alternative embodiment of the device. In particular, the proximal engaging element may not be connected with the tubing compartment. Rather, the proximal engaging element (65) that is associated with the proximal element connector (41) may freely slide along the central wire 10 as seen in Figure 26A. In some embodiments, the inner diameter of the proximal element connector (41) is lager than the outer diameter of the central wire (10), and thus the proximal engaging element (65) that is associated with the proximal element connector 41 can slide freely on the central wire (10). Further, a connecting tubing may not be in need in at least some embodiments. Figure 26B shows a closed-status of the embodiment shown in Figure 26A where the central wire (10) is pulled proximally and thus the distal engaging element 90 moves toward the proximal engaging element (65). This will shorten the space (or distance) between the two engaging elements. This feature would enable the device to engage a clot as illustrated in the next figure.

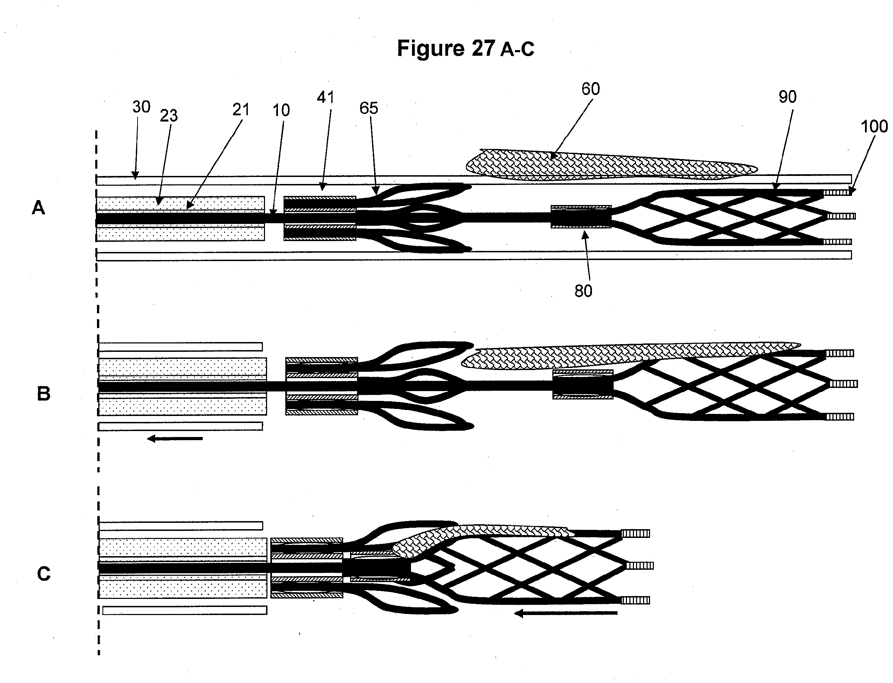

Figure 27 shows an illustrative embodiment of removing a clot or occlusion from a body lumen (e.g. blood vessel), especially using the device shown in Figure 26. In certain embodiments, the device may be used to engage a clot or occlusion and remove the same from the body lumen as follows:
(A) A microcatheter (30), with guidance of a guide wire is first navigated to an area where an occlusion occurs in a body lumen. The microcatheter may be positioned distal to the proximal end of the clot or occlusion. Depending on hardness, size, location and shape of the clot or occlusion, the microcatheter may penetrate the clot (or occlusion), or pass by the clot without substantially disturbing the clot. Then the tubing compartment and the engaging compartment may be delivered to the occlusion through the microcatheter. The proximal expendable element is separated from the distal expendable element. When placing the device, the tip of the proximal engaging element is placed proximal to the clot or occlusion.
(B) The microcatheter is unsheathed, and the engaging compartment and part of the tubing compartment are exposed. An operator can adjust the device so that the proximal engaging element may be proximal to the clot, and the distal engaging element may be distal to the clot, or at least passing part of the clot, if the clot is substantially long. In some embodiments, the clot would be held at least in part by the distal engaging element at this stage as seen in the figure.
(C) An operator can further adjust the position of the distal engaging elements. For example, the operator can proximally pull the central wire that is connected to the distal engaging element. Then the clot would be moved proximally due to the friction between the distal engaging element and the clot. The distance between the two engaging elements is shortened. When the clot contacts the proximal engaging element, the clot can be grabbed or engaged in and/or between the two engaging elements. In the meantime, the engaging compartment and the clot is future pulled to near the microcatheter tip. To ensure the engagement, the operator may lock the position of the distal engaging element and pull the device.

In certain embodiments, the process illustrated in Figure 27 can be performed as follows: while performing the process demonstrated in Figure 26, there may be friction between the microcatheter lumen and the proximal engaging element when the device is unsheathed, and the proximal engaging element is pulled away from the distal engaging element. The distance between the two engaging elements may be maximized or substantially extended once the two elements are out of the microcatheter. An operator may not need to adjust the positions of the two engaging elements after deployment. An operator can pull the distal engaging element proximally through the pusher tubing which is connected to the central wire. Therefore, the distance between the two components will be shortened to engage the clot. In certain occasions, the tip of microcatheter may be used to prevent the proximal engaging element moving backward. When the operator feels resistance while pulling the pusher tubing/central wire proximally, it may indicate that the clot is engaged in and/or between the two engaging elements and the proximal engaging element is also stopped by the tip of microcatheter. At this point the device can be locked and removed from the body lumen. Therefore the clot or occlusion can be removed easily and with high efficiency.

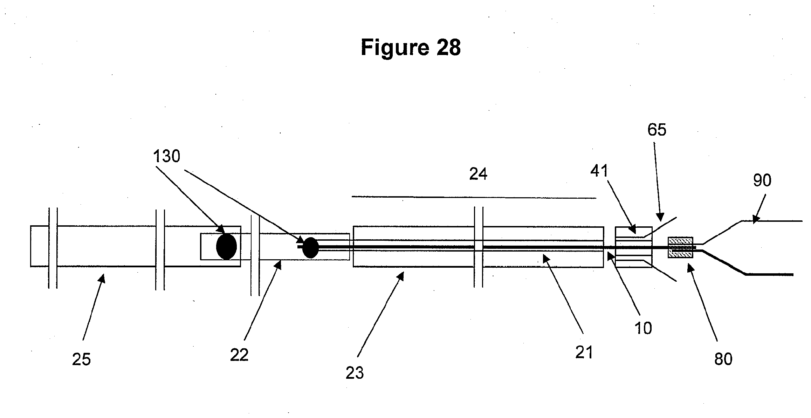

Figure 28 illustrates another illustrative embodiment of a system comprising the device illustrated in Figure 26. Alternatively, the device illustrated in Figure 20 can be used in this system of Figure 28. In some embodiments, the proximal engaging element (65) is around the central wire (10), and thus it can slide freely between the distal engaging element 90 and the distal tip of the pusher tubing (140). In addition, the distal engaging element 90 may be connected to the central wire (10). The central wire (10) can extend to the proximally. The central wire 10 may be joined with at least one of the pusher tubings (e.g. proximal pusher tubing 25, middle pusher tubing 22, inner pusher tubing 21, and outer pusher tubing 23). Therefore, unlike the embodiment illustrated in Figure 24, the central wire 10 does not necessarily extend to the proximal end of the device. Instead, it may be connected with any of the tubing elements, and be controlled along with the connected tubing. In some embodiments, the outer pusher tubing (23) may be flexible and lubricious, and placed close to the distal end of the system so that it may allow the device to pass through tortuous path in the lumen. Further, in some other embodiments, a thin inner pusher tubing (21) may be present in the system, and this inner pusher tubing (21) may be flexible yet can increase the pushability of the system. In still some other embodiments, a middle pusher tubing (22) with certain flexibility may be present in the system. In still some other embodiments, a distal and/or proximal pusher tubing(s) (24, 25) that is/are relatively stiff may be present in the system so that the pushability of the system may be further improved. One or more pusher tubings can be connected with adhesives to each other, and also with other parts including a central wire (10). In still some other embodiments, a single pusher tubing with variable stiffness can be used in this device to replace multiple pusher tubings and junctions. This tubing can be made by grinding the tubing into different wall thicknesses, or through spiral cutting to ensure the distal end being more flexible and proximal end being more stiff.

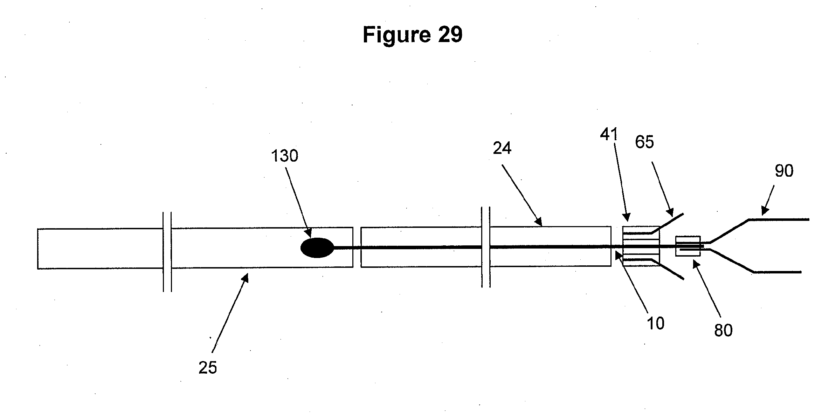

Figure 29 illustrates an alternative embodiment of a system in which a distal pusher tubing and a proximal pusher tubing are present. In this embodiment, the central wire (10) may not extend to the proximal end of the system. Similar to the example shown in Figure 28, the central wire is connected to one or more of the tubing elements, and controlled via the connected tubing elements. On top of the connection tubing, there is a distal pusher tubing. In some embodiments, at least some part of the distal pusher tubing is flexible to ensure the device being able to navigate through tortuous path. In other embodiments, at least some part of the distal pusher tubing may be lubricious and flexible, and thus it also enhances pushability of the devise. In certain embodiments, the proximal end of the pusher tubing can be made from a stiff/firm tubing to enhance the device pushability.

Figure 30A illustrates an alternative embodiment of a device. Unlike the embodiment illustrated in Figure 20, this device comprises a push-able and pull-able proximal engaging element. In this design, however, the distal tip of the proximal engaging element is modified. The distal end of the proximal engaging element may be bent inward or smoothed as shown in Figure 30 which may further ensure that the tips are atraumatic to the inner wall of the artery. Thus it can be pulled and pushed in the artery lumen with much lower risk of damaging the artery wall. Figure 30B illustrates a closed-status of the device shown in Figure 30A where the distance between the proximal and distal engaging elements is minimized.

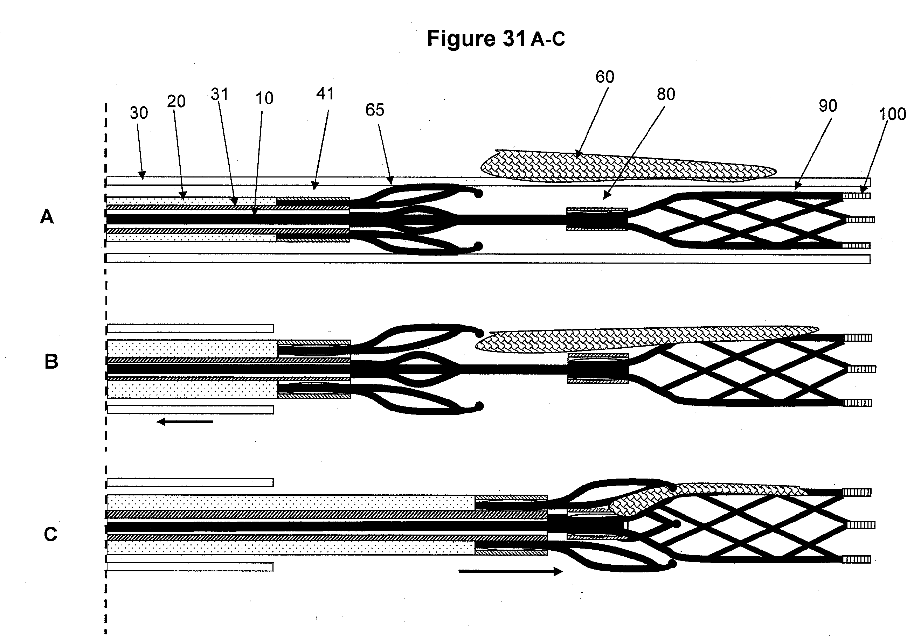

Figure 31 shows an illustrative embodiment of removing a clot or occlusion from a body lumen (e.g. blood vessel), especially using the device illustrated in Figure 30. The clot retrieval mechanism illustrated in this figure is largely similar to that in Figure 21 and 22.

The device according to some embodiments of the present invention has significant advantages. For example, during the operation if the clot is not in and/or between the two engaging elements, or one or both the engaging elements is either too distal or too proximal to the clot, an operator can position one or both of the engaging elements to ensure the engagement. Especially, in the embodiments illustrated in Figures 31, the proximal engaging element is adjusted to a desired position by pulling or pushing through the connection tubing. Because the proximal element has an atraumatic round tip and can be pushed forward to engage the clot with the distal element, the procedure/technique may avoid the need to pull the clot for a distance with distal engaging element only, which may further reduce the possibility of losing the clot during the procedure. Therefore, such a device can engage with the clot using the two engaging elements, and stabilize the same until the clot is removed from the body lumen. This independent control of the two engaging elements allow very fine adjustment of the device during the procedure, and therefore it is highly useful, especially in settings when precise positioning in treatment is necessary (e.g. stroke treatment in brain).

Figure 32A illustrates an alternative embodiment of a device. In this particular embodiment, the proximal engaging element has a basket-like feature. There is no relatively sharp end on this proximal engaging element (65) because the distal ends of the proximal engaging element are connected to a distal connector (140) of the proximal engaging element via a connecting wire (150) of proximal engaging element. Therefore, when the proximal engaging element is compressed, the distal end thereof will move backward and form a smooth distal end as shown in Figure 32B. This smooth ends is atraumatic to the artery inner lumen. This proximal engaging element is pull-able and push-able in body lumen in at least some embodiments. In certain embodiments, the distal end of the proximal engaging element is thinner than the other part thereof. Thus, when a clot is pulled or pushed in and/or between the two engaging elements, the distal portion of the proximal engaging element may buckle, or invert as seen in Figure 32B so that engagement of the clot by the proximal engaging element would not be hindered by additional association between the proximal engaging element 65 and the distal connector of proximal element (140).

Figure 33A illustrates a further alternative embodiment of a device. In this particular embodiment, the proximal engaging element has a basket-like feature. However, unlike the embodiment illustrated in Figure 32, the distal end of the proximal engaging element 65 is directly jointed with the distal connector of proximal component (140). This device also has no sharp end on the proximal engaging element, and thus further reduces any risks damaging the body lumen. In this design, the material used in a proximal portion of the proximal engaging element could either be same as, or different from, the other portion thereof. In certain embodiments, the distal portion of the proximal engaging element may be made of a relatively flexible element than the other portion thereof. Accordingly, when a clot is pulled against the proximal engaging element, the distal portion of the proximal engaging element may be buckle or invert so that the proximal engaging element can better engage with the clot as seen in Figure 33B.

Figure 34A illustrates a further alternative embodiment of a device. This device also comprises additional features for further ensuring the safety of the device. This design may comprise the proximal engaging element having a basket like feature. The distal portion of the proximal element is jointed to the proximal portion of the proximal element through soft/flexible connectors (e.g. a segment connect 160) which allow buckling while the distal portion if compressed backward. This proximal engaging element is pull-able and push-able, e.g. by controlling the tubing compartment associated with the proximal engaging element. There is no sharp end in the proximal engaging element, and therefore it is atraumatic to the artery wall. Moreover, in this design, the proximal engaging element may be made of more than one material, one of which is softer than the other. Therefore, it is easy to buckle or invert at least the distal portion of the proximal engaging element when engaging with the clot (see Figure 34B).

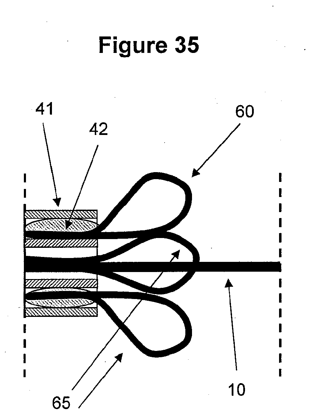

Figure 35 illustrates a further alternative embodiment of the proximal portion a device. This device also comprises additional features for further ensuring the safety of the device. This design comprises the proximal engaging element with round distal ends to make the tips more atraumatic to the vessel. Thus the proximal engaging element is pull-able and push-able, e.g. by controlling the tubing compartment associated with the proximal engaging element.

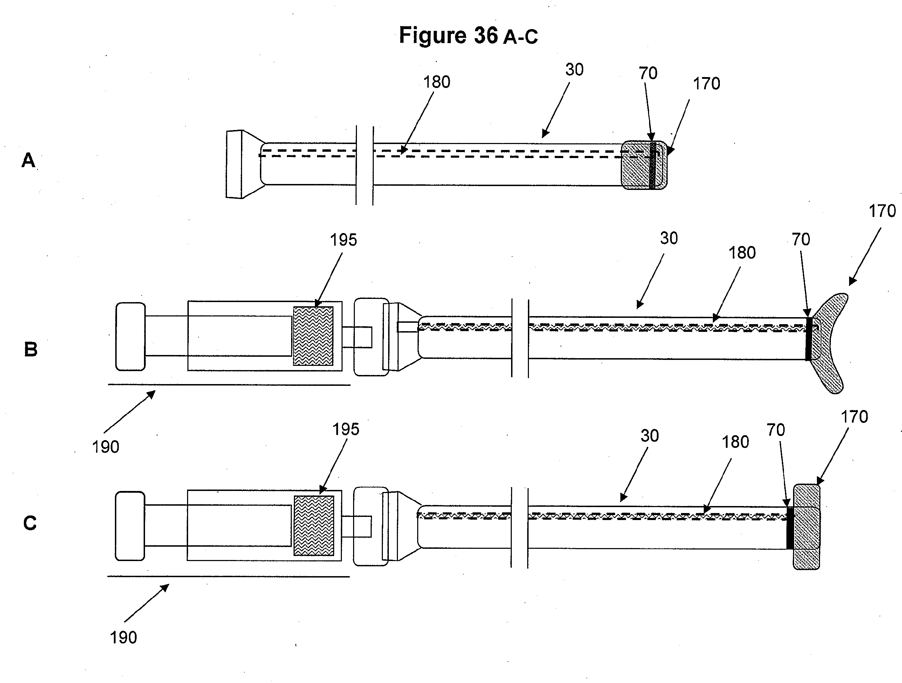

The following Figures 36-45 provide further alternative embodiments where a proximal element for engaging (and removing) a clot is located at the distal tip or end of a microcatheter. In some embodiments, this engaging element may be attached at the distal tip of the microcatheter. In some other embodiments, a distal tip or end of a microcatheter itself is shaped and configured to act as a proximal engaging element. In other words, the proximal engaging element is an integral part of the microcatheter.

In some embodiments, a proximal engaging element, which may be separately attached to or an integral part of the microcatheter, can change a shape and/or size during a retrieval process or when needed. For instance, after a retrieval device is placed in the desired position, i.e. fully or partially passing a clot, the retrieval device may be inserted to a microcatheter. The microcatheter can then be pulled proximally to unsheathe the device. When the distal tip of the microcatheter reaches at about the proximal end of the clot, the distal tip of the microcatheter is maneuvered to change into an open/funnel shape. While holding the microcatheter stable, the retriever device can be pulled back with the clot that may be partially engaged with the engaging element of the retriever until the clot is substantially or fully engaged (or captured) between the distal tip of the microcatheter (i.e. the proximal engaging element) and the engaging element of the retriever (i.e. the distal engaging element). By locking the retrieval device and the microcatheter at the proximal end of the devices, the device and the microcatheter together with the engaged clot can be pulled out from the lumen, e.g. an artery

In certain embodiments where the microcatheter distal tip provides a proximal engaging element, it may simplify a design of a retrieval device, and consequently the manufacturing process thereof. Instead of two separate engaging elements, e.g. as illustrated in an embodiment of Figure 20, the retrieval device may need only one engaging element, i.e. a distal engaging element, as another element acting as a proximal engaging element can be provided from the microcatheter.

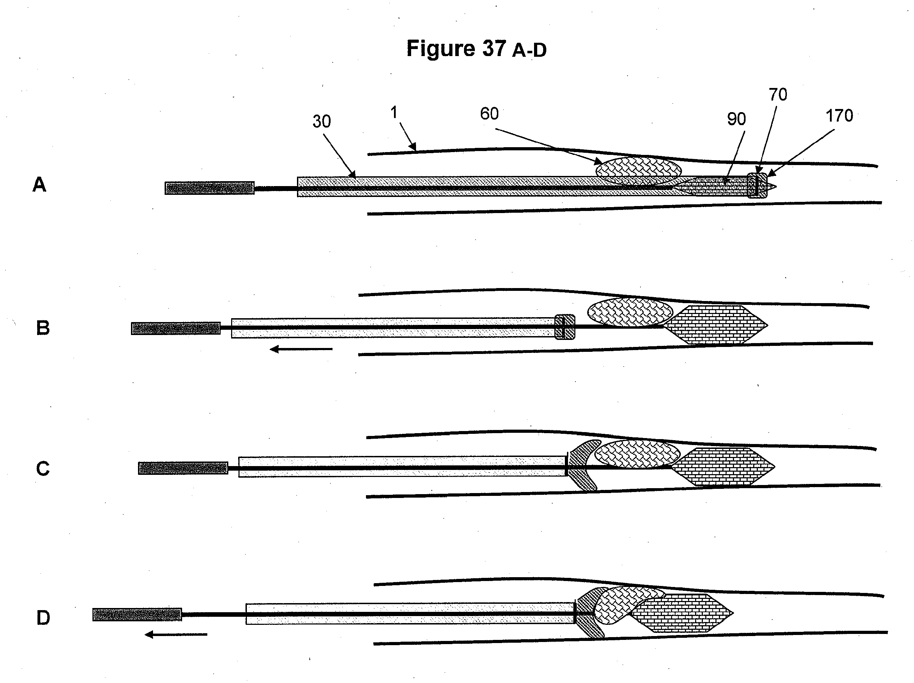

Figure 36 illustrates an embodiment of a device where an inflatable or engaging element such as a balloon is attached at the distal end/tip of the microcatheter. The shape and size of the inflatable or engaging element (170) may be controlled by its inflation and deflation. According to some embodiments, the inflatable or engaging element (170) can be inflated e.g. by injecting a liquid (195) (e.g. a saline solution) with a syringe 190 into the inflatable or expandable (170) element so as to form a desired shape. The injected liquid can be transferred to the inflatable or expandable element (170) through an injection channel 180 in certain some embodiments. The shape and size of the inflatable or engaging element (170) after being inflated can be varied. For instance, when a preformed balloon is attached at the distal tip of the microcatheter, it can be shaped into any preformed shape, e.g. a funnel-like form, when inflated, as seen in Figure 36B. Alternatively, merely the surface area of the inflatable or engaging element (170) can be increased upon inflation as seen in Figure 36C. This transformable proximal engaging element provided by the microcatheter can engage the clot alone or in combination with other elements (e.g. a surface of the body lumen, the microcatheter, the tubing compartment, the proximal engaging element, the distal engaging element, and any combinations thereof) with high efficiency (see, e.g. Figure 33 and 37).

Figure 37 shows a mechanism to engage and remove a clot using the device of Figure 36. During the retrieval procedure, an operator may insert the microcatheter (30) into a body lumen (e.g. an artery) until the distal tip passes into or through the clot (60). The retrieval device may be delivered to the occlusion position along with the microcatheter, or through the microcatheter once the microcatheter is at the position. In certain embodiments, the retrieval device may be pushed until its engaging element 90 may pass at least portion of the clot as seen in Figure 37A. While holding the retrieval device stable, the operator may pull the microcatheter backward (i.e. proximally) until the microcatheter's distal tip is at about the proximal end of the clot, and the engaging element 90 of the retrieval device is exposed, and expanded to its relaxed or open state (Figure 37B). Therefore, the clot may be located between the distal engaging element 90 and the distal tip of the microcatheter (i.e. the proximal engaging element 170). Then, the operator may expand the transformable distal tip of the microcatheter 170 to transform the tip into a funnel shape at the proximal end of the clot (Figure 37C). While holding the microcatheter stable, the operator may pull the distal engaging element 90 back so that the clot may be moved backward and engaged between the distal engaging element 90 and the distal tip of the microcatheter (170). The operator then can fix the microcatheter and the retrieval device and pulling them out of the artery which results in the removal of the clot.

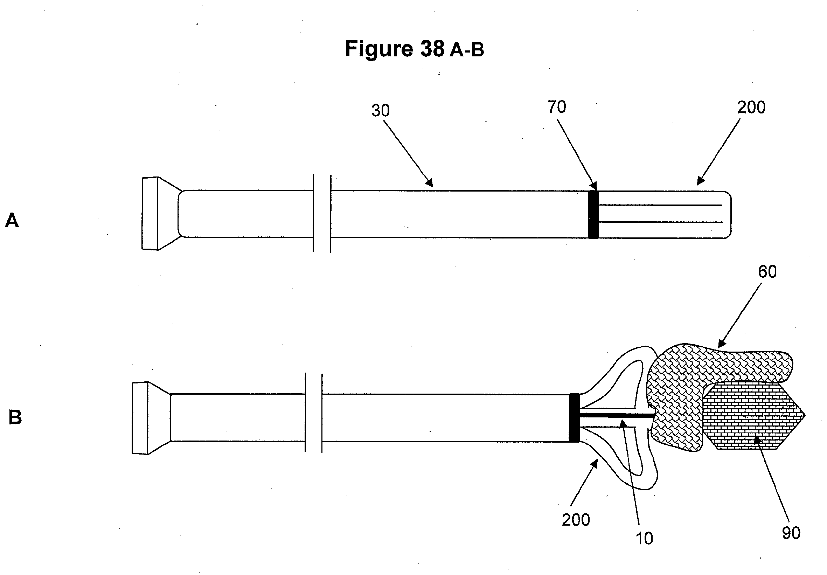

Figure 38 illustrates another embodiment of a device where a distal tip of a microcatheter forms an engaging element, more particularly a proximal engaging element. In this particular embodiment, a distal tip of the microcatheter (200) is cut, e.g. by a laser cutting, so that it can transform into a funnel shape when compressed by the clot (60) and/or the distal engaging element 90 during a retrieval procedure (Figure 38B). In certain embodiments, the cutting process ends before reaching the very end of the microcatheter distal tip so that the very distal tip of the microcatheter is still closed.

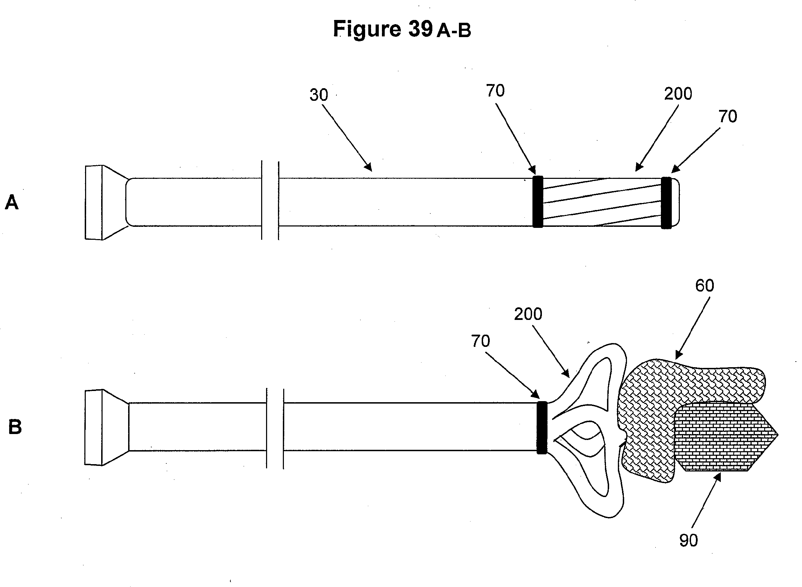

Figure 39 illustrates a further alternative of the embodiment in which the distal tip (200) of the microcatheter receives spiral cuts. Similar in the device of Figure 38, the microcatheter's distal tip will be shaped into, e.g. a funnel form, when compressed during a retrieval procedure (Figure 39B). In certain embodiments, the cutting process ends before reaching the very end of the microcatheter tip so that the very distal tip of the microcatheter is still closed.

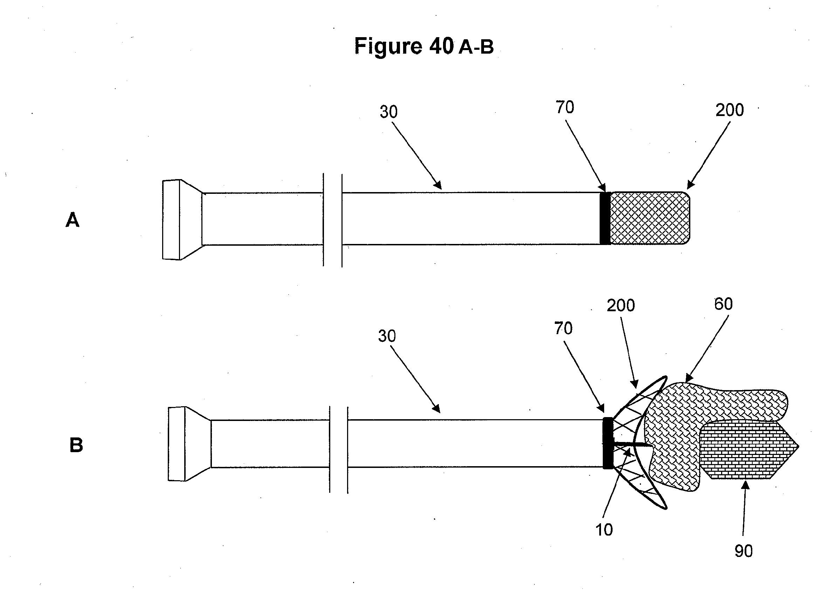

Figure 40 illustrates still another embodiment wherein the distal tip of the microcatheter is formed into a braid structure. Alternative, as described elsewhere in the application, a separate braid structure can be attached at the microcatheter's distal tip. The braid structure (200) that is attached at or formed in the microcatheter may be a metal braid with or without a plastic coating. Upon engaging a clot along with the distal engaging element 90, the braid structure (200) will be compressed and shaped into, e.g. a funnel form, so that the clot is held between the distal engaging element 90 and the braid structure (200).

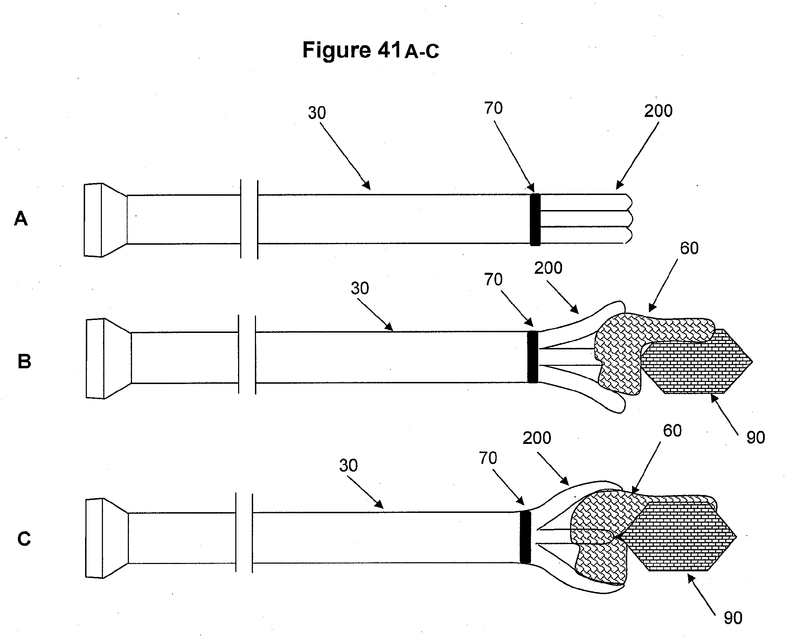

Figure 41 illustrates still another embodiment wherein the cutting of the microcatheter's distal tip is through to the very end. Therefore, as seen in the figure, the very end of the microcatheter's distal tip is not closed. Upon pulling a clot backward with the distal engaging component 90, the tip of the microcatheter is compressed into, e.g. a funnel shape (spitted), and the clot is held between the distal engaging component 90 of the retrieval device and the distal tip of the microcatheter (200). Figure 41B and C show two different shapes of the opening tip of the microcatheter.

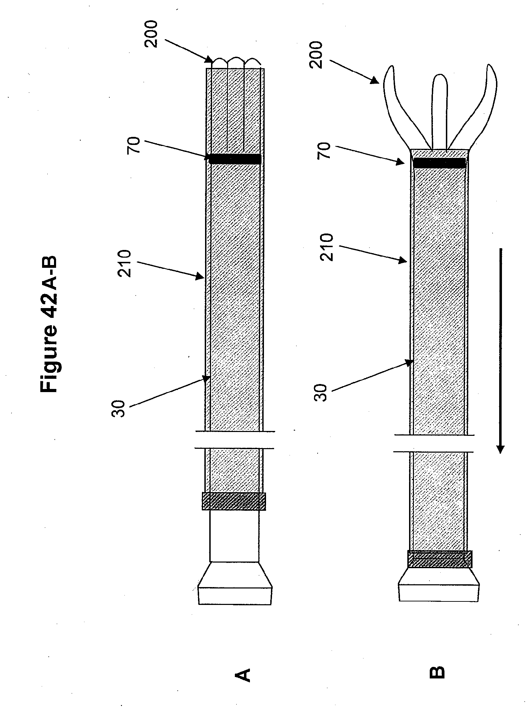

In certain embodiments, the proximal engaging element may comprise a portion of the distal end of the microcatheter comprising a microcather tip and a layer of thin tubing that covers the microcatheter tip. In some embodiments, at least part of the proximal engaging element is configured to change a shape when a layer of thin tubing is removed. An example of such embodiments is presented in Figure 42. Figure 42 illustrates a device in which a layer of outer sheath is applied to a microcatheter. In certain embodiments, a distal tip of the microcatheter used as an engaging element may comprise a pre-shaped structure or a shape-memory structure. The outer sheath (210) can hold the microcatheter's distal tip (200) so it can pass a clot prior to the engagement. When the microcatheter is pulled proximal to the clot, by pulling the outer sheath proximally, the tip opens to its pre-shaped, e.g. funnel shape. Alternatively, a shape-memory material can be used in the microcatheter's distal tip (200) so that it can form a certain shape when uncovered from the outer sheath.

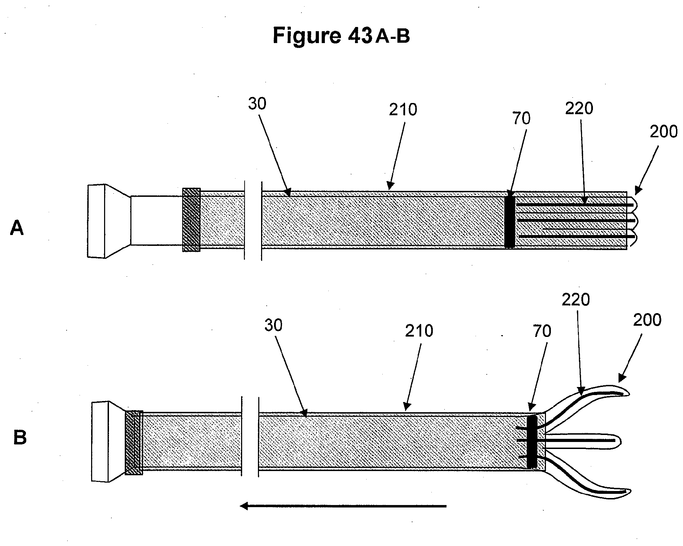

Figure 43 illustrates still another embodiment of a device in which a wire is used to provide a certain shape at the microcatheter's distal tip. This design illustrated in Figure 43B shows the microcatheter's distal tip (200) having a wire (220), e.g. an elastic wire, embedded in the tip to ensure that the tip will expand to its pre-set shape after being pulled out of the outer sheath (210).

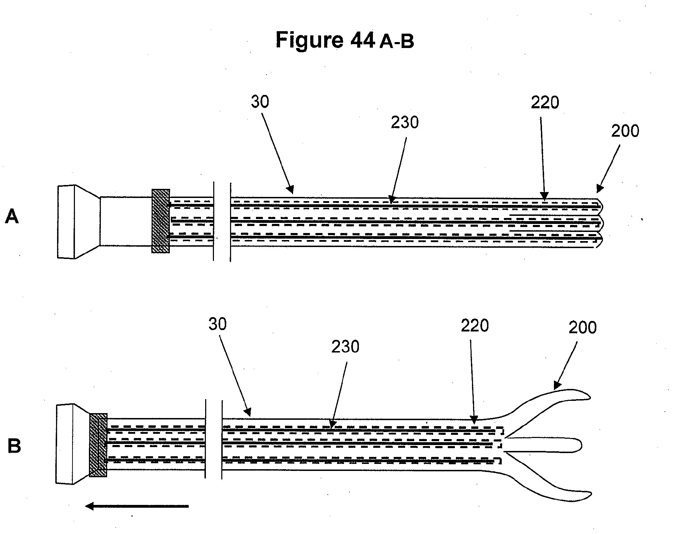

Figure 44 illustrates still another embodiment of a device. In this particular embodiment, a pre-shaped funnel tip structure (sliced) is placed at the tip of a microcatheter, and the microcatheter's tip is held by wires (220) running through lumen(s) in the inner channel (230) of the microcatheter wall. The wires can hold the microcatheter's tip straight or unfolded until the tip passes the clot prior to the engagement. When the microcatheter is pulled proximal to the clot, by pulling the wires proximally, the tip opens to its pre-shaped, e. g. funnel shape.

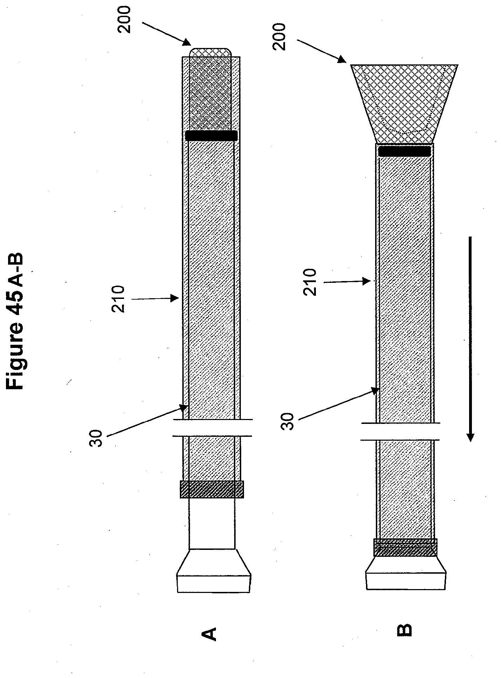

Figure 45 illustrates still another embodiment of a device where a braid structure and an outer sheath are adopted together. A pre-shaped braid structure is placed at the microcatheter's distal tip, and a layer of an outer sheath holds the tip straight so the can be advanced in the circulation up to or through the clot prior to the engagement. When the microcatheter is - positioned proximal to clot, by pulling the outer sheath proximally, the braid tip opens to its pre-shaped, e.g. funnel shape.

## Claims

1. A device for use in a body lumen comprising:
a microcatheter (30) for delivery of the device;
a tubing compartment (20, 31);
a central wire (10); and
an engaging compartment, wherein
the tubing compartment (20,31) comprises a pusher tubing (20) and a connecting tubing (31) connected to each other; and
the engaging compartment comprises:
a proximal engaging element (65) comprising a plurality of wires or made from a tubing by a laser cutting technique, said proximal engaging element (65) being open at a distal end thereof, the distal end of said proximal engaging element (65) being bent inward so that tips of the proximal engaging element are atraumatic to an inner wall of the body lumen; and
a distal engaging element (90) comprising a plurality of wires or struts, said distal engaging element (90) being self-expandable and a proximal end thereof placed between an outer connector (82) and the central wire (10),
wherein a portion of the occlusion (60) can be captured between the proximal end of the distal engaging element (90) and the distal end of the proximal engaging element (65) when the distance between the distal engaging element (90) and the proximal engaging element (65) is shortened;
wherein the proximal end of the distal engaging element (90) is fixed at the distal end of the central wire (10), said central wire (10) extending to a proximal end of the device, whereby the position of the distal engaging element (90) in the body lumen is controlled by movement of the central wire (10); and
wherein the occlusion (60) may be engaged:
(a) between of the distal engaging element (90) and the wall of the body lumen; and
(b) between the proximal engaging element (65) and the distal engaging element (90);
(c) between the tubing compartment (21,30) and the wall of the body lumen,
(d) between the microcatheter (30) and the proximal engaging element (65),
(e) between the proximal engaging element (65) and the wall of the body lumen, and
(f) between a tip of the microcatheter (30) and the same time between the proximal engaging element (65) and the distal engaging element (90).

2. The device according to Claim 1 wherein the proximal engaging element (65) is fixed with the tubing compartment (20, 31) extending to a proximal end of the device, thereby a location of the proximal engaging element (65) in the body lumen is controlled by movement of the tubing compartment (20, 31).

3. The device according to Claim 1, wherein the tubing compartment (20, 31) is made from one piece of tubing with variable stiffness such that a distal end of the tubing compartment (20, 31) is soft and flexible so the device can pass tortuous anatomy while a proximal end of the tubing compartment is stiff to enhance pushability of the device.

4. The device according to Claim 1, wherein the central wire (10) comprises a wire, a cable, or a braid.

5. The device according to Claim 1, wherein the proximal engaging element (65) comprises a distal end facing the distal engaging element (90), said distal end of the proximal engaging element (65) being rounded, or smoothed.

6. The device according to Claim 1, wherein the distal end of the proximal engaging element (65) is configured not to be in direct contact with a surface of the body lumen.

7. The device according to Claim 1, wherein the positions of the distal engaging element (90) and the proximal engaging element (65) are configured to lock such that the movement of the two engaging elements after being locked is synchronized and their introduction into a microcatheter (30) is in succession.

8. The device according to Claim 1, wherein the proximal engaging element (65) and the distal engaging element (90) are configured to be independently controlled.

9. The device according to Claim 1, wherein the distal engaging element (90) has an open-end at a distal end.

10. The device according to Claim 1, wherein the distal engaging element (90), the outer connector (82), and the central wire (10) are connected by joining media.

11. The device according to Claim 1, wherein the joint media (81) comprises a clip, clasp or fastener.

12. The device according to Claim 1, further comprising a proximal engaging element connector (41).

13. The device according to Claim 12, wherein the proximal engaging element connector (41) comprises joining media (42) and an outer proximal element connector (43).

14. The device according to Claim 13, wherein a proximal end of the proximal engaging element (65) is placed between the outer proximal element connector (43) and a tip of the connecting tubing (31).

15. The device according to Claim 1, comprising markers (100) for identifying the location of the device.

16. The device according to Claim 1, wherein the proximal engaging element (65) is configured to be pushed forward to engage the occlusion (60) with the distal engaging element (90).

## Patentansprüche

1. Vorrichtung zur Verwendung in einem Körperlumen, umfassend:
einen Mikrokatheter (30) zur Zuführung der Vorrichtung;
eine Rohrkammer (20, 31);
einen zentralen Draht (10); und
eine Eingriffskammer,
wobei die Rohrkammer (20, 31) ein Pusherrohr (20) und ein damit verbundenes Verbindungsrohr (31) umfasst; und
die Eingriffskammer umfasst:
ein proximales Eingriffselement (65), das eine Vielzahl von Drähten umfasst oder das aus einem Rohr durch eine Laserschneidtechnik hergestellt ist, wobei das proximale Eingriffselement (65) an einem distalen Ende davon offen ist, wobei das distale Ende des proximalen Eingriffselements (65) nach innen gebogen ist, so dass die Spitzen des proximalen Eingriffselements für eine Innenwand des Körperlumens atraumatisch sind; und
ein distales Eingriffselement (90), das eine Vielzahl von Drähten oder Streben umfasst, wobei das distale Eingriffselement (90) selbstexpandierbar ist und ein proximales Ende davon zwischen einem äußeren Verbinder (82) und dem zentralen Draht (10) angeordnet ist,
wobei ein Abschnitt der Okklusion (60) zwischen dem proximalen Ende des distalen Eingriffselements (90) und dem distalen Ende des proximalen Eingriffselements (65) erfasst werden kann, wenn der Abstand zwischen dem distalen Eingriffselement (90) und dem proximalen Eingriffselement (65) verkürzt wird;
wobei das proximale Ende des distalen Eingriffselements (90) am distalen Ende des zentralen Drahtes (10) befestigt ist, wobei sich der zentrale Draht (10) zu einem proximalen Ende der Vorrichtung erstreckt, wodurch die Position des distalen Eingriffselements (90) im Körperlumen durch die Bewegung des zentralen Drahtes (10) gesteuert wird; und
wobei in die Okklusion (60) eingegriffen werden kann:
(a) zwischen dem distalen Eingriffselement (90) und der Wand des Körperlumens; und
(b) zwischen dem proximalen Eingriffselement (65) und dem distalen Eingriffselement (90);
(c) zwischen der Rohrkammer (21, 30) und der Wand des Körperlumens,
(d) zwischen dem Mikrokatheter (30) und dem proximalen Eingriffselement (65),
(e) zwischen dem proximalen Eingriffselement (65) und der Wand des Körperlumens und
(f) zwischen einer Spitze des Mikrokatheters (30) und gleichzeitig zwischen dem proximalen Eingriffselement (65) und dem distalen Eingriffselement (90).

2. Vorrichtung nach Anspruch 1, wobei das proximale Eingriffselement (65) mit der Rohrkammer (20, 31) fest verbunden ist, die sich bis zu einem proximalen Ende der Vorrichtung erstreckt, wodurch eine Position des proximalen Eingriffselements (65) in dem Körperlumen durch Bewegung der Rohrkammer (20, 31) gesteuert wird.

3. Vorrichtung nach Anspruch 1, wobei die Rohrkammer (20, 31) aus einem Stück Rohr mit variabler Steifigkeit hergestellt ist, so dass ein distales Ende der Rohrkammer (20, 31) weich und flexibel ist, so dass die Vorrichtung gewundene Anatomie passieren kann, während ein proximales Ende der Rohrkammer steif ist, um die Schiebbarkeit der Vorrichtung zu verbessern.

4. Vorrichtung nach Anspruch 1, wobei der zentrale Draht (10) einen Draht, ein Kabel oder eine Litze umfasst.

5. Vorrichtung nach Anspruch 1, wobei das proximale Eingriffselement (65) ein dem distalen Eingriffselement (90) zugewandtes distales Ende umfasst, wobei das distale Ende des proximalen Eingriffselements (65) abgerundet oder geglättet ist.

6. Vorrichtung nach Anspruch 1, wobei das distale Ende des proximalen Eingriffselements (65) so ausgelegt ist, dass es nicht in direktem Kontakt mit einer Oberfläche des Körperlumens steht.

7. Vorrichtung nach Anspruch 1, wobei die Positionen des distalen Eingriffselements (90) und des proximalen Eingriffselements (65) so ausgelegt sind, dass sie verriegelt werden, so dass die Bewegung der beiden Eingriffselemente nach der Verriegelung synchronisiert wird und ihre Einführung in einen Mikrokatheter (30) nacheinander erfolgt.

8. Vorrichtung nach Anspruch 1, wobei das proximale Eingriffselement (65) und das distale Eingriffselement (90) so ausgelegt sind, dass sie unabhängig gesteuert werden können.

9. Vorrichtung nach Anspruch 1, wobei das distale Eingriffselement (90) ein offenes Ende an einem distalen Ende aufweist.

10. Vorrichtung nach Anspruch 1, wobei das distale Eingriffselement (90), der äußere Verbinder (82) und der zentrale Draht (10) durch Verbindungsmedien verbunden sind.

11. Vorrichtung nach Anspruch 1, wobei das Verbindungsmaterial (81) einen Cip, eine Klammer oder ein Befestigungselement umfasst.

12. Vorrichtung nach Anspruch 1, ferner umfassend einen proximalen Eingriffselement-Verbinder (41).

13. Vorrichtung nach Anspruch 12, wobei der proximale Eingriffselement-Verbinder (41) Verbindungsmedien (42) und einen äußeren proximalen Element-Verbinder (43) umfasst.

14. Vorrichtung nach Anspruch 13, wobei ein proximales Ende des proximalen Eingriffselements (65) zwischen dem äußeren proximalen Element-Verbinder (43) und einer Spitze des Verbindungsrohrs (31) angeordnet ist.

15. Vorrichtung nach Anspruch 1, umfassend Markierungen (100) zum Identifizieren der Position der Vorrichtung.

16. Vorrichtung nach Anspruch 1, wobei das proximale Eingriffselement (65) ausgelegt ist, um nach vorne geschoben zu werden, um in die Okklusion (60) mit dem distalen Eingriffselement (90) einzugreifen.

## Revendications

1. Dispositif pour utilisation dans une lumière corporelle, comprenant :
un microcathéter (30) pour placer le dispositif ;
un compartiment de tubage (20, 31) ;
un fil central (10) ; et
un compartiment d'engagement,
dans lequel
le compartiment de tubage (20, 31) comprend un tuyau à poussoir (20) et un tuyau de raccordement (31) connectés l'un à l'autre ; et
le compartiment d'engagement comprend :
un élément d'engagement proximal (65) comprenant une pluralité de fils ou réalisé à partir d'un tuyau par une technique de découpe au laser, ledit élément d'engagement proximal (65) étant ouvert à une extrémité distale de celui-ci, l'extrémité distale dudit élément d'engagement proximal (65) étant recourbée vers l'intérieur de telle sorte que des pointes de l'élément d'engagement proximal sont inoffensives pour une paroi intérieure de la lumière corporelle ; et
un élément d'engagement distal (90) comprenant une pluralité de fils ou d'entretoises, ledit élément d'engagement distal (90) étant auto-expansible et une extrémité proximale de celui-ci étant placée entre un connecteur externe (82) et le fil central (10),
dans lequel une portion de l'occlusion (60) peut être capturée entre l'extrémité proximale de l'élément d'engagement distal (90) et l'extrémité distale de l'élément d'engagement proximal (65) lorsque la distance entre l'élément d'engagement distal (90) et l'élément d'engagement proximal (65) est raccourcie ;
dans lequel l'extrémité proximale de l'élément d'engagement distal (90) est fixée à l'extrémité distale du fil central (10), ledit fil central (10) s'étendant jusqu'à une extrémité proximale du dispositif, de sorte que la position de l'élément d'engagement distal (90) dans la lumière corporelle est contrôlée par le mouvement du fil central (10) ; et
dans lequel l'occlusion (60) peut être engagée :
(a) entre l'élément d'engagement distal (90) et la paroi de la lumière corporelle ; et
(b) entre l'élément d'engagement proximal (65) et l'élément d'engagement distal (90) ;
(c) entre le compartiment de tubage (21, 30) et la paroi de la lumière corporelle,
(d) entre le microcathéter (30) et l'élément d'engagement proximal (65),
(e) entre l'élément d'engagement proximal (65) et la paroi de lumière corporelle, et
(f) entre une pointe du microcathéter (30) et en même temps entre l'élément d'engagement proximal (65) et l'élément d'engagement distal (90).

2. Dispositif selon la revendication 1, dans lequel l'élément d'engagement proximal (65) est fixé au compartiment de tubage (20, 31) s'étendant jusqu'à une extrémité proximale du dispositif, de sorte qu'une position de l'élément d'engagement proximal (65) dans la lumière corporelle est commandée par le mouvement du compartiment de tubage (20, 31).

3. Dispositif selon la revendication 1, dans lequel le compartiment de tubage (20, 31) est réalisé à partir d'une seule pièce de tube ayant une épaisseur variable de telle sorte qu'une extrémité distale du compartiment de tubage (20, 31) est douce et flexible de sorte que le dispositif peut traverser une anatomie tortueuse tandis qu'une extrémité proximale du compartiment de tubage est épaisse pour augmenter la capacité de poussée du dispositif.

4. Dispositif selon la revendication 1, dans lequel le fil central (10) comprend un fil, un câble ou une tresse.

5. Dispositif selon la revendication 1, dans lequel l'élément d'engagement proximal (65) comprend une extrémité distale faisant face à l'élément d'engagement distal (90), ladite extrémité distale de l'élément d'engagement proximal (65) étant arrondie, ou adoucie.

6. Dispositif selon la revendication 1, dans lequel l'extrémité distale de l'élément d'engagement proximal (65) est configurée pour ne pas être en contact direct avec la surface de la lumière corporelle.

7. Dispositif selon la revendication 1, dans lequel les positions de l'élément d'engagement distal (90) et de l'élément d'engagement proximal (65) sont configurées pour se verrouiller de telle sorte que le mouvement des deux éléments d'engagement après leur verrouillage est synchronisé et que leur introduction dans un microcathéter (30) se fait en succession.

8. Dispositif selon la revendication 1, dans lequel l'élément d'engagement proximal (65) et l'élément d'engagement distal (90) sont configurés pour être commandés indépendamment.

9. Dispositif selon la revendication 1, dans lequel l'élément d'engagement distal (90) comprend une extrémité ouverte à une extrémité distale.

10. Dispositif selon la revendication 1, dans lequel l'élément d'engagement distal (90), le connecteur externe (82), et le fil central (10) sont connectés par des moyens de jonction.

11. Dispositif selon la revendication 1, dans lequel les moyens de jonction (81) comprennent un clip, un fermoir ou une attache.

12. Dispositif selon la revendication 1, comprenant en outre un connecteur d'élément d'engagement proximal (41).

13. Dispositif selon la revendication 12, dans lequel le connecteur d'élément d'engagement proximal (41) comprend un moyen de jonction (42) et un connecteur d'élément proximal externe (43).

14. Dispositif selon la revendication 13, dans lequel une extrémité proximale de l'élément d'engagement proximal (65) est placée entre le connecteur d'élément proximal externe (43) et une pointe du tube de connexion (31).

15. Dispositif selon la revendication 1, comprenant des marqueurs (100) pour identifier la position du dispositif.

16. Dispositif selon la revendication 1, dans lequel l'élément d'engagement proximal (65) est configuré pour être poussé en avant pour s'engager sur l'occlusion (60) par l'élément d'engagement distal (90).
